# EUROPEAN PATENT APPLICATION

(11) **EP 4 111 912 A2**
(43) Date of publication of application: **04.01.2023**
(21) Application number: 22167272.8
(22) Date of filing: 08.04.2022
(51) Int. Cl.: A47C 27/08, A61B 5/11, A61B 5/024, A61B 5/08

(54) **PNEUMATIC HAPTIC DEVICE HAVING ACTUATION CELLS FOR PRODUCING A HAPTIC OUTPUT OVER A BED MATTRESS**

(30) Priority: 04.06.2021 US 202117339738
(71) Applicant: Apple Inc., Cupertino, CA 95014 (US)
(72) Inventor: TADELE, Wegene H., Cupertino, CA, 95014 (US); HERRERA, Antonio F., Cupertino, CA, 95014 (US); BRANDT, Riley E., Cupertino, CA, 95014 (US); ZHANG, Yingjun, Pudong, Shanghai, 200122 (CN); LOR, Jason, Cupertino, CA, 95014 (US)
(74) Representative: Lang, Johannes

(57) **Abstract**

Embodiments are directed to a pressure mapping system that includes a sensing pad configured for placement between a user and a bed. The sensing pad can include an array of actuation cells where each actuation cell in the array of actuation cells is configured to actuate in response to a fluid being introduced into the actuation cell. The sensing pad can also include an array of pressure sensors where each pressure sensor in the array of pressure sensors is configured to output pressure measurements. The pressure mapping system can also include a control system that is configured to receive the pressure measurements from the array of pressure sensors, generate a pressure map for the user based on the set of pressure measurements and a position of the pressure sensing cells on the sensing pad, and actuate a subset of the array of actuation cells based on the pressure map.

## Description

### CROSS-REFERENCE TO RELATED APPLICATIONS

This application is a continuation in part of U.S. Patent Application No. 17/020,585, filed September 14, 2020, which is a nonprovisional of and claims the benefit under 35 U.S.C. § 119(e) of U.S. Provisional Patent Application No. 62/902,811, filed September 19, 2019, each of the contents of which are incorporated herein by reference as if fully disclosed herein.

### FIELD

The described embodiments relate generally to devices and systems for mapping a user's position or posture and providing haptic outputs. More particularly, the described embodiments relate to an in-bed haptic device having actuation cells and a control system configured to introduce pressurized air (or another fluid) into the actuation cells to map a pressure profile of a user and/or provide haptic outputs.

### BACKGROUND

Electronic devices may have one or more input mechanism for sensing parameters of a user positioned on a bed or other support surface and output mechanisms that provide tactile outputs to a user of the device. In general, it may be beneficial for electronic devices to identify a position or posture of a user and/or provide tactile outputs to users while they are in bed. Some traditional electronic devices may provide tactile feedback to users in bed, but the types of tactile feedback that can be provided are limited and devices can cause discomfort to users.

### SUMMARY

Embodiments of the systems, devices, methods, and apparatuses described in the present disclosure are directed to an in-bed device having an array of sensing devices that are used to map a user's contact with the bed, and use the mapping to provide a haptic feedback to the user, identify the user, determine a posture of the user, measure a physiological parameter or the user, among other functions.

Embodiments described herein include an in-bed haptic system including a sensing pad configured for placement between a user and a bed. The sensing pad can include an array of actuation cells, where each actuation cell in the array of actuation cells is configured to actuate in response to a fluid being introduced into each actuation cell, and an array of pressure sensors, where each pressure sensor in the array of pressure sensors is configured to output pressure measurements. The in-bed haptic system can include a control system that is configured to receive the pressure measurements from the array of pressure sensors, generate a pressure map for the user based on the pressure measurements and a position of each pressure sensor on the sensing pad, and actuate actuation cells of the array of actuation cells based on the pressure map.

Embodiments also include a pressure mapping system that includes a sensing pad configured to be integrated with a user support structure. The sensing pad can include an array of actuation cells, each actuation cell in the array of actuation cells configured to actuate in response to a fluid being introduced into an actuation cell and an array of pressure sensors, where each pressure sensor in the array of pressure sensors coupled with the actuation cell of the array of actuation cells. Each pressure sensor can be configured to measure a fluid pressure within a corresponding actuation cell, and output one or more signals indicative of the measured fluid pressure for each actuation cell. The sensing pad can include a control system configured to receive the one or more signals from the array of pressure sensors, determine that a user is contacting the user support structure, and in response to determining the user contact, generate a pressure map for the user based on the received one or more signals. The control system can identify the user as a specific user based on the pressure map.

Embodiments further include methods of operating a pressure sensing haptic device to identify a posture of a user. The methods can include receiving a set of pressure measurements from an array of pressure sensors positioned on a user support structure, and generating a pressure map for the user based on the set of pressure measurements and positions of pressure sensors within the array of pressure sensors. The methods can also include comparing the pressure map to a set of stored pressure maps, the set of stored pressure maps comprising posture maps that are each associated with a specific posture, and identifying the posture of the user based on the comparison of the pressure map to the set of stored pressure maps.

### BRIEF DESCRIPTION OF THE DRAWINGS

The disclosure will be readily understood by the following detailed description in conjunction with the accompanying drawings, wherein like reference numerals designate like structural elements, and in which:
FIG. 1 shows an example environment for using an in-bed haptic device;
FIG. 2A shows the example in-bed haptic device and the control system of FIG. 1;
FIGS. 2B-2C show an example haptic output provided by the in-bed haptic device of FIG. 1;
FIGS. 3A-3C show an example haptic output provided by the in-bed haptic device of FIG. 1;
FIG. 4 shows an example block diagram of a control system that is fluidly coupled to an in-bed haptic device;
FIG. 5 shows a flowchart of an example method for providing a haptic output using an in-bed haptic device;
FIG. 6 shows a sensor array embodiment of the in-bed haptic device and control system of FIG. 1;
FIG. 7 shows an embodiment of a user positioned on an in-bed haptic device;
FIG. 8 shows a flowchart of an example method for identifying a user and or a posture of a user of a haptic device;
FIGS. 9A-9G show an example in-bed haptic device;
FIGS. 10A-10E show an example in-bed haptic device;
FIGS. 11A-11B illustrate an example connection interface that includes multiple tubular members that are fluidly coupled to individual passages of an in-bed haptic device;
FIGS. 12A-12B illustrate an example connection interface in which passages defined in the in-bed haptic device are extended to form the connection interface;
FIGS. 13A-13B illustrate an example arrangement of example components of a control system;
FIG. 14 shows an example of an in-bed haptic device and control system;
FIGs. 15A-15B illustrate examples of actuation cells that can be integrated into an in-bed device;
FIGs. 16A-16C illustrate cross-sectional views of example actuation cells;
FIGs. 17A-17B illustrate example actuation cells that can be implemented in an in-bed device; and
FIG. 18 shows a sample electrical block diagram of an electronic device that may incorporate and/or be connected to an in-bed haptic device.

The use of cross-hatching or shading in the accompanying figures is generally provided to clarify the boundaries between adjacent elements and also to facilitate legibility of the figures. Accordingly, neither the presence nor the absence of cross-hatching or shading conveys or indicates any preference or requirement for particular materials, material properties, element proportions, element dimensions, commonalities of similarly illustrated elements, or any other characteristic, attribute, or property for any element illustrated in the accompanying figures.

Additionally, it should be understood that the proportions and dimensions (either relative or absolute) of the various features and elements (and collections and groupings thereof) and the boundaries, separations, and positional relationships presented therebetween, are provided in the accompanying figures merely to facilitate an understanding of the various embodiments described herein and, accordingly, may not necessarily be presented or illustrated to scale, and are not intended to indicate any preference or requirement for an illustrated embodiment to the exclusion of embodiments described with reference thereto.

### DETAILED DESCRIPTION

Reference will now be made in detail to representative embodiments illustrated in the accompanying drawings. It should be understood that the following description is not intended to limit the embodiments to one preferred embodiment. To the contrary, it is intended to cover alternatives, modifications, and equivalents as can be included within the spirit and scope of the described embodiments as defined by the appended claims.

The following disclosure relates to a system for providing haptic outputs. The system may include an in-bed haptic device having an array of actuation cells. Actuation cells of the array of actuation cells may be configured to actuate (e.g., expand, contract, or otherwise change shape) in a predetermined sequence to provide haptic outputs. The haptic outputs may be provided in response to detected inputs at the in-bed haptic device or another electronic device, or in association with notifications, alerts, or other outputs at the in-bed haptic device or another electronic device.

As used herein, the term "haptic output" may be used to refer to a device output that is tactilely perceptible along the user's body as series localized impulses that are generally dynamic in nature. As used herein, the term "localized impulse" may be used to refer to a brief force acting along a portion of a user's body. As described herein, a portion of the haptic output may be created by one or more actuation cells that are inflated and deflated to produce a tactilely perceptible impulse.

In some cases, the actuation cells are configured to actuate in response to fluid being introduced into and/or removed from the actuation cells. As used herein, "fluid" may be used to refer to substances that have no fixed shape, which allows them to flow, including air, other gasses, liquids, and combinations thereof. The in-bed haptic device may include an enclosure that is configured to be placed beneath a user during use. In some cases, the in-bed haptic device may be positioned between a mattress and a user during use. The in-bed haptic device may provide haptic outputs along a top external surface that may be perceived tactilely (e.g., sensed through touch) by the user. The in-bed haptic device may be sufficiently thin and/or flexible so that the in-bed haptic device, when positioned in a bed beneath the user, does not cause discomfort.

The actuation cells may cause deformation and/or displacement of the top external surface to provide haptic outputs and/or portions thereof. Actuation of a particular actuation cell may cause deformation and/or displacement of a corresponding portion of the top external surface. As used herein, "deformation" may be used to refer to changing a shape or contour of a surface, element, or a portion thereof, and "displacement" may be used to refer to moving a surface, element, or a portion thereof from a first position to one or more additional positions relative to one or more additional surfaces, elements, or portions thereof. Generally, when a surface, element, or portion thereof is deformed, at least a portion of it is displaced. For example, if the top external surface is deformed, a portion of the top external surface will necessarily be moved relative to another portion of the top external surface. However, displacement does not necessarily require deformation. For example, the entire top external surface may be displaced relative to other components of the in-bed haptic device without changing a shape or contour of the top external surface.

In some cases, deformation caused by the actuation cells may be local deformation. As used herein, "local deformation" or "locally deforming" may be used to refer to deforming a localized portion of a surface or element while not deforming one or more other portions of the surface or element (e.g., portions surrounding the localized portion). Multiple actuation cells may cooperate to locally deform the top external surface of the in-bed haptic device. Multiple portions of a surface or element may be locally deformed at the same time, during overlapping time periods, and/or at different times (e.g., sequentially).

In some cases, multiple actuation cells may cooperate to produce a haptic output. Multiple portions of the top external surface may be displaced and/or deformed by actuation of multiple different actuation cells to produce a haptic output. In some cases, multiple different portions of the top external surface are displaced and/or deformed according to a pattern to provide a haptic output. In some cases, actuation of the actuation cells in a predetermined sequence may cause the external surface to displace and/or deform according to an actual or simulated randomized pattern (e.g., no pattern is discernable). For example, the predetermined sequence may simulate a pattern of falling raindrops. In some cases, actuation of the actuation cells in a predetermined sequence may cause the external surface to displace and/or deform according to an ordered (e.g., non-random) pattern. For example, the predetermined sequence may simulate a wave moving at least partially across the top external surface of the in-bed haptic device.

Each actuation cell of the array of actuation cells may include one or more bladders defining an interior volume and configured to inflate and/or deflate to cause the actuation cells to actuate to provide haptic outputs and/or portions of haptic outputs. For example, inflation of the one or more bladders may cause the actuation cell to expand and deflation of the one or more bladders may cause the actuation cell to contract. Each bladder may be configured to inflate in response to a pressurized air (or another fluid) being introduced into the interior volume and/or deflate in response to a pressurized air being removed from the interior volume. In some cases, each actuation cell of the array of actuation cells is configured to expand in a direction that is substantially transverse to the top external surface, thereby increasing a thickness of a region of the in-bed haptic device corresponding to the cell.

The in-bed haptic device may be fluidly coupled to a control system that is configured to introduce pressurized air into the bladders of the array of actuation cells and/or remove pressurized air from the bladders of the array of actuation cells to provide haptic outputs. The control system may include one or more reservoirs configured to facilitate rapid inflation and/or deflation of bladders. In some cases, the control system includes one or more high pressure reservoirs containing air having a pressure that is higher than atmospheric pressure and/or one or more vacuum reservoirs containing air having a pressure that is lower than atmospheric pressure. A valve array of the control system may be configured to selectively fluidly couple each cell (e.g., the bladder(s) of each actuation cell) to one or more reservoirs. For example, the control system (e.g., a processing unit of the control system) may cause a valve between a bladder of an actuation cell and the high pressure reservoir to open to inflate the bladder. Similarly, the control system may cause a valve between a bladder of an actuation cell and the vacuum reservoir to deflate the bladder.

In some cases, a processing unit may provide signals to the control system and/or the in-bed haptic device to provide haptic outputs. The processing unit may be a component of the in-bed haptic device, the control system, or another electronic device operably coupled to the in-bed haptic device and/or the control system. The in-bed haptic device, the control system, and/or another device operably connected to the processing unit may include one or more input devices (e.g., contact sensors, force sensors, audio sensors, biometric sensors, images sensors, light sensors and the like) configured to detect inputs that are used by the processing unit to determine to provide haptic outputs and/or the types of haptic outputs to provide. The processing unit may determine a haptic output to provide in response to one or more detected inputs, and may control various components of the control system and/or the in-bed haptic device (e.g., valves, pumps, etc.) to provide the haptic output.

The inputs received by the processing unit may be used to determine triggers for providing haptic outputs. Triggers may include user conditions that indicate whether a user is asleep or awake, present or not present, snoring or not snoring, and the like. User conditions may be determined by analyzing signals from input devices (e.g., contact sensors, force sensors, audio sensors, biometric sensors, images sensors, light sensors, and the like). For example, the processing unit may determine user conditions by determining breathing information (e.g., instantaneous breathing rate, average breathing rate, maximum breathing rate, minimum breathing rate), user movement or presence information (e.g., using a force or pressure sensor or transducer), heart information (e.g., instantaneous heart rate, average heart rate, maximum heart rate, minimum heart rate) determined from contact sensors, force sensors, audio sensors, biometric sensors, and the like. Triggers may also include raw inputs received from the in-bed haptic device and/or other devices, outputs provided by a device (e.g., audio outputs, video outputs, haptic outputs, alerts or alarms, and the like), and other conditions (e.g., time of day, temperature, humidity, weather, and other environmental conditions). In response to detecting or determining one or more triggers, the processing unit may determine one or more haptic outputs to be provided and cause the control system and/or the in-bed haptic device to provide the haptic output(s).

In some cases, the system can be operated in a detection mode to determine a profile, position, posture, or other physiological parameter of the user. The device can include an array of sensors that are configured to generate a pressure map along a user's body as the user contacts the device. The pressure map can be used to identify a specific user, determine a posture or position of a user, differentiate between multiple users positioned on the device, and/or identify subsets of actuation cells or other sensors that a user is contacting. In some cases, the location and/or posture/position data can be used to provide haptic feedback to the user. For example, a subset of actuation cells can be selected for providing a haptic feedback based on the position of the user on the device. In other examples, the pressure map can be used to analyze a pressure distribution across a portion of the user's body such as their torso section. The pressure distribution can be analyzed and used to provide feedback to the user about their posture, which can include making recommendations to a user to change their posture or position. In some cases, the pressure distribution can be used to locally control the stiffness of the device along different portions of a user's body, for example by increasing the pressure in individual actuation cells. The local stiffness changes can be used to help change a user's pressure distribution and/or encourage a user to change position. In some cases, the pressure map can be used to select a subset of sensors for performing physiological measurements of a user, which can include monitoring heart rate, other cardiac activity, breathing, and so on.

In some cases, the actuation cells may be individually addressed. As used herein, "individually addressed" may be used to refer to actuation cells that may be controlled independently of one another. In some cases, each actuation cell may be controlled independently of all other actuation cells of the array of actuation cells. In some cases, actuation cells may be grouped into cell groups, and the actuation cells in the cell group are controlled together, but independently of other cell groups and/or actuation cells.

The control system may include one or more pumps configured to establish and maintain the pressure(s) of the reservoirs. The control system may include a pressurizing pump configured to increase the pressure and/or maintain the increased pressure in the high pressure reservoir. The control system may include a vacuum pump configured to decrease the pressure and/or maintain the decreased pressure in the vacuum reservoir. Using the reservoirs for inflating and/or deflating the bladders of individual actuation cells of the in-bed haptic device may allow the individual cells to be inflated and/or deflated more rapidly than using pumps to inflate and/or deflate the bladders. The pumps of the control system can pressurize or depressurize the reservoirs over a long period of time in advance of providing haptic outputs to "charge" the reservoirs so that more rapid pressure changes may occur. In addition, using the reservoirs for inflating and/or deflating the bladders of the in-bed haptic device may reduce potential disturbances (e.g., sound, vibration, and the like) that would be created by using a pump for inflation and/or deflation. For example, the pumps of the control system can pressurize or depressurize the reservoirs before use of the in-bed haptic device (e.g., while a user is not present), thereby minimizing disturbances to users.

The control system may include one or more connectors that fluidly couple the control system (e.g., the reservoirs) to the in-bed haptic device. The in-bed haptic device may include passages that fluidly couple actuation cells and corresponding bladder(s) of the array of actuation cells to one or more connectors. The connectors and the passages may cooperate to define fluid paths that fluidly couple the reservoirs to the actuation cells of the array of actuation cells. As noted above, one or more valves (e.g., a valve array) are operable to control the fluid coupling between the reservoirs and the actuation cells. For example, a valve may be opened to fluidly couple one or more bladders to a reservoir via one or more connectors so that fluid may flow between the bladders and the reservoir. Similarly, a valve may be closed to terminate a fluid coupling so that fluid may not flow between the bladders and the reservoir. The valves may be positioned at any suitable location along the fluid path between a reservoir and one or more bladders, including within the control system, connector(s), passages, or actuation cells. The connectors of the control system allow the control system and the in-bed haptic device to be positioned separately from one another. In some cases, the control system may be located far enough away from the in-bed haptic device (and the user), such as in another room, that potential disturbances (e.g., sounds, vibrations, and the like) produced by the control system may not disturb a user.

In some cases, a thickness of the in-bed haptic device is much smaller than its length and/or width. For example, the thickness (e.g., a distance between a top surface and a bottom surface) of the in-bed haptic device may be less than approximately ten percent, five percent, or even one percent of the width of the in-bed haptic device. The thickness of the in-bed haptic device may be less than approximately one percent, one half of one percent, or even one tenth of one percent of the length of the in-bed haptic device. The dimensions of the in-bed haptic device may provide numerous advantages, including increasing a flexibility of the in-bed haptic device, improving comfort of the in-bed haptic device, and/or reducing a user-perceptibility of the in-bed haptic device during use.

The term "attached," as used herein, may be used to refer to two or more elements, structures, objects, components, parts or the like that are physically affixed, fastened, and/or retained to one another. The term "coupled," as used herein, may be used to refer to two or more elements, structures, objects, components, parts or the like that are physically attached to one another, operate with one another, communicate with one another, are in electrical connection with one another, and/or otherwise interact with one another. Accordingly, while elements attached to one another are coupled to one another, the reverse is not required. As used herein, "operably coupled" may be used to refer to two or more devices that are coupled in any suitable manner for operation and/or communication, including wiredly, wirelessly, or some combination thereof. As used herein, "fluidly coupled" may be used to refer to two or more volumes, elements structure, objects components, parts, or the like that are in fluid communication with one another such that fluid may flow between or among the two or more volumes, elements structure, objects components, parts, or the like.

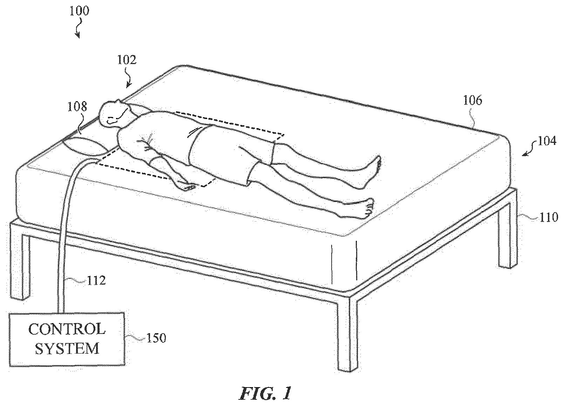

These and other embodiments are discussed with reference to FIGS. 1-18. However, those skilled in the art will readily appreciate that the detailed description given herein with respect to these figures is for explanatory purposes only and should not be construed as limiting.

FIG. 1 shows an example environment for using an in-bed haptic device 100 (shown in phantom). As shown in FIG. 1, the in-bed haptic device 100 may be positioned beneath a user 102 as the user is in a bed 104. The in-bed haptic device 100 may be adapted to provide haptic outputs to users. The haptic outputs provided by the in-bed haptic device 100 may be provided in response to detected inputs at the in-bed haptic device or another electronic device, or in association with notifications, alerts, or other outputs at the in-bed haptic device or another electronic device.

In some cases, the in-bed haptic device 100 is adapted to be positioned between a user 102 and a mattress 106 of a bed 104. The in-bed haptic device 100 may be sufficiently thin and/or flexible so that the in-bed haptic device, when positioned in a bed 104 beneath a user 102, does not cause discomfort. In some cases, a thickness of the in-bed haptic device 100 is much smaller than its length and/or width. For example, the thickness (e.g., a distance between a top surface and a bottom surface) of the in-bed haptic device 100 may be less than approximately ten percent, five percent, or even one percent of the width of the in-bed haptic device. The thickness of the in-bed haptic device 100 may be less than approximately one percent, one half of one percent, or even one tenth of one percent of the length of the in-bed haptic device. The dimensions of the in-bed haptic device 100 may provide numerous advantages, including increasing a flexibility of the in-bed haptic device, improving comfort of the in-bed haptic device, and/or reducing a user-perceptibility of the in-bed haptic device during use.

The in-bed haptic device 100 may include an array of actuation cells configured to expand and/or contract to provide haptic outputs and/or portions thereof. Actuation cells of the array of actuation cells may be configured to actuate (e.g., expand, contract, or otherwise change shape) in a predetermined sequence to provide haptic outputs. In some cases, the actuation cells include one or more bladders configured to inflate and/or deflate to actuate the actuation cells. The in-bed haptic device 100 may include an enclosure that is configured to be placed beneath a user 102 during use. The in-bed haptic device 100 may provide haptic outputs along a top external surface of the enclosure that may be perceived tactilely by the user 102. In some cases, the top external surface of the enclosure defines a modifiable contour, and actuation of the actuation cells modifies the modifiable contour to provide haptic outputs.

The in-bed haptic device 100 can be an array of sensors that are configured to detect one or more physiological parameters of the user 102. The array of sensors can include pressure sensors that can be used to generate a pressure map of the user, which can be used to determine a profile, position, posture, or other parameters of the user. In some cases, the sensors can be integrated with the actuation cells to measure a fluid pressure within each actuation cell. Additionally or alternatively, the array of sensors can include other types of sensors, such as temperature sensors, proximity sensors, capacitive sensors, piezo-electric sensors, strain-based sensors, acoustic sensors, and vibration sensors, among others.

In various embodiments, the in-bed haptic device 100 and/or the control system 150 may be connected to a companion device configured to provide triggers for providing haptic outputs, control signals, and other information. The companion device may be any suitable electronic device, including sleep monitors, wearable electronic devices, timekeeping devices, health monitoring or fitness devices, portable computing devices, mobile phones (including smart phones), tablet computing devices, digital media players, virtual reality devices, audio devices (including earbuds and headphones), and the like.

In some cases, the haptic outputs may correspond to inputs, outputs, alerts, or notifications at the in-bed haptic device or another electronic device. As another example, a haptic output may correspond to an alert or notification received at the in-bed haptic device 100 or a connected device, such as a phone call, a received message, a push notification, or the like. In some cases, an alert may correspond to a biometric or similar characteristic of the user 102. For example, the haptic output may be provided in response to a heart rate, breathing rate, or other biometric detected by the in-bed haptic device 100 or another device falling above or below a predetermined threshold.

In various embodiments, the haptic outputs may be provided while a user is awake or asleep. In some cases, the in-bed haptic device 100 or another device may detect whether a user is awake or asleep and may provide, modify, or cease a haptic output in response to the determination.

The in-bed haptic device 100 may be positioned above or beneath a mattress 106 and/or bed frame 110 of the bed 104. The in-bed haptic device 100 may be positioned above or beneath bedding of the bed 104, including a mattress protector, sheets, blankets, and the like. In some cases, the in-bed haptic device 100 is positioned above the mattress 106 and beneath at least some layers of bedding. For example, the in-bed haptic device 100 may be positioned above a mattress protector, but beneath a bottom sheet of the bedding. In some cases, the in-bed haptic device 100 includes adhesive along one or more surfaces so that the in-bed haptic device 100 may be attached or coupled to the mattress 106 or bedding of the bed (e.g., a mattress protector). In some cases, the in-bed haptic device 100 is placed between approximately 10 and 40 centimeters from a pillow 108. The in-bed haptic device 100 may be centered in a sleeping area of the user 102.

Although the in-bed haptic device 100 is primarily described in relation to the bed 104, it will be appreciated that the devices described herein can be implemented in a variety of other use cases. For example, the devices described herein can be configured for use with furniture, cars, hospital beds, cribs, or other suitable structures, which may be generally referred to herein as "user support structures." The haptic device can be used to determine a pressure map of a user who is sitting on or otherwise engaged with various user support structures. As described herein, the pressure map can be used to identify a particular user, or determine a posture, position, or other parameter of a user. In some cases, the pressure map data can be used to provide feedback to the user, which can include providing alerts or suggestions to the user about their posture or position, providing haptic feedback to the user, and/or providing other alerts or feedback to the user. In some cases, the identity of a user, based a pressure map, can be used to control a support structure that the user is engaged with, such as adjusting a car seat, adjusting a hospital bed, or other user support structure.

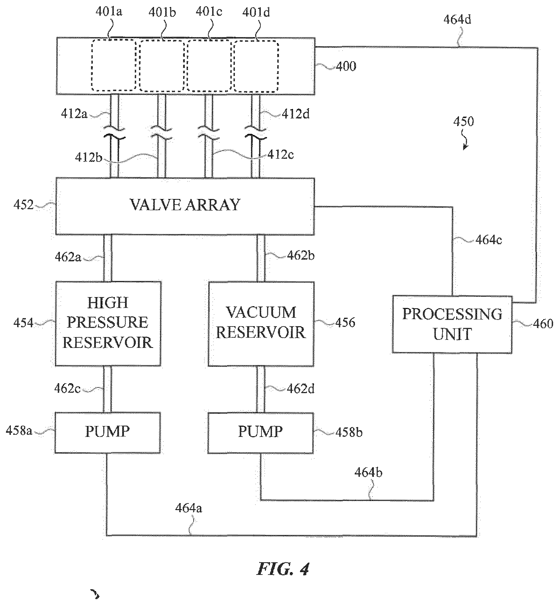

The in-bed haptic device 100 may be operably and/or fluidly coupled to a control system 150.The control system 150 may be configured to introduce pressurized air into one or more actuation cells (e.g., into an interior volume of the bladder(s)) of the in-bed haptic device 100 and/or remove pressurized air from one or more actuation cells (e.g., from an interior volume of the bladder(s)) of the in-bed haptic device in a predetermined sequence to provide haptic outputs. As discussed in more detail below with respect to FIG. 4, the control system 150 may cause haptic outputs provided by the in-bed haptic device 100 in response to receiving signals from a processing unit (e.g., a processing unit of the control system 150 or a processing unit of another electronic device).

The control system 150 and/or the in-bed haptic device 100 may include one or more connectors 112 that fluidly couple the control system to the in-bed haptic device. The connector(s) 112 of the control system 150 allow the control system and the in-bed haptic device 100 to be positioned separately from one another. In some cases, the control system 150 may be located far enough away from the in-bed haptic device 100 (and the user 102), such as in another room, that potential disturbances (e.g., sounds, vibrations, and the like) produced by the control system 150 may not disturb the user 102. The connectors 112 can also couple the array of sensors to the in-bed haptic device to the control system 150. In some cases, the array of sensors can be configured to wirelessly transmit sensor data to the control system 150 and/or other electronic device such as a companion device described herein.

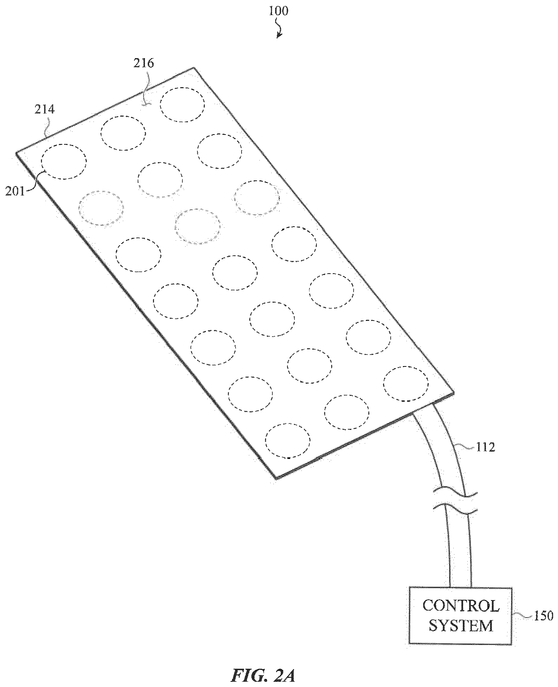

FIG. 2A shows the in-bed haptic device 100 and the control system 150 of FIG. 1. As noted above, the in-bed haptic device 100 may include an array of actuation cells 201 (shown in phantom in FIG. 2A) configured to actuate (e.g., expand, contract, or otherwise change shape) to provide haptic outputs and/or portions thereof. The in-bed haptic device 100 may include an enclosure 214 other external layer that at least partially surrounds the actuation cells 201 and/or other components of the in-bed haptic device 100. The enclosure 214 may contain and/or protect the actuation cells 201 and/or other components of the in-bed haptic device 100. In some cases, the enclosure 214 is flexible. One or more surfaces of the enclosure 214 may include an adhesive or a high-friction material(s) configured to maintain the in-bed haptic device 100 in place.

The enclosure 214 may define a top external surface 216 of the in-bed haptic device 100. In various embodiments, haptic outputs provided by the in-bed haptic device 100 may be provided at and/or through the top external surface 216. In some cases, the top external surface 216 defines a modifiable contour, and actuation of the actuation cells modifies the modifiable contour to provide haptic outputs. In various embodiments, the enclosure 214 may not fully enclose or surround the components of the in-bed haptic device 100. For example, the enclosure 214 may be defined by top and bottom layers with components of the in-bed haptic device 100 in between, in which case the sides of the in-bed haptic device 100 may not be enclosed by the enclosure 214. In some cases, the enclosure 214 has an open top (e.g., the enclosure 214 does not enclose at least a portion of the top of the in-bed haptic device). For example, the array of actuation cells may define at least a portion of the top external surface 216.

The in-bed haptic device 100 may include an array of actuation cells 201 configured to expand and/or contract in predetermined sequences to provide haptic outputs. Each actuation cell 201 of the array of actuation cells may include one or more bladders defining an interior volume and configured to inflate and/or deflate to cause the actuation cells to actuate to provide haptic outputs and/or portions of haptic outputs. For example, inflation of the one or more bladders may cause the actuation cell 201 to expand and deflation of the one or more bladders may cause the actuation cell to contract. Each bladder may be configured to inflate in response to a pressurized air (or another fluid) being introduced into the interior volume and/or deflate in response to a pressurized air being removed from the interior volume. In some cases, each actuation cell 201 of the array of actuation cells is configured to expand in a direction that is substantially transverse to the top external surface 216, thereby increasing a thickness of a region of the in-bed haptic device 100 corresponding to the cell 201. The in-bed haptic device 100 is shown in FIG. 2A as having twenty-one actuation cells 201 ordered in a two-dimensional array. This is an example and is not meant to be limiting. The in-bed haptic devices described herein may include any suitable number of actuation cells arranged in any suitable way.

As noted above, each actuation cell 201 may be individually addressed. In some cases, each actuation cell 201 may be controlled independently of all other actuation cells of the array of actuation cells. For example, providing a haptic output may include inflating a first actuation cell 201 while maintaining an adjacent actuation cell 201 in an uninflated state. In some cases, actuation cells 201 may be grouped into cell groups, and the actuation cells in the cell group are controlled together, but independently of other cell groups and/or actuation cells. To facilitate the independent control of the actuation cells 201, each actuation cell may be independently fluidly coupled (or capable of being fluidly coupled) to the control system 150. For example, each actuation cell 201 may be fluidly coupled to the control system 150 by one or more fluid paths defined in the in-bed haptic device 100, the connector 112, and/or the control system 150. In some cases, as discussed in more detail below with respect to FIG. 4, one or more valves may be positioned along the fluid path between each actuation cell 201 and the control system 150 to control the fluid coupling.

The actuation cells 201 may cause deformation and/or displacement of the top external surface 216 to provide haptic outputs and/or portions thereof. Actuation of a particular actuation cell 201 may cause deformation and/or displacement of a corresponding portion of the top external surface 216. For example, an actuation cell 201 may inflate (partially or fully) to provide a first portion of a haptic output (e.g., a first localized impulse) and may deflate (partially or fully) to provide a second portion of a haptic output (e.g., a first localized impulse). In addition, an actuation cell 201 may remain static (e.g., deflated, partially inflated, or fully inflated) during a haptic output (e.g., between inflation or deflation or while one or more other actuation cells inflate or deflate).

As noted above, as used herein, the term "haptic output" may be used to refer to a device output that is tactilely perceptible along the user's body as series localized impulses that are generally dynamic in nature, and the term "localized impulse" may be used to refer to a brief force acting along a portion of a user's body. A haptic output or a portion thereof may be provided by an actuation (e.g., an inflation or deflation) of one or more actuation cells 201. In some cases, the duration of an actuation of an actuation cell 201, such as an inflation period (e.g., a duration that an actuation cell is inflating) or a deflation period (e.g., a duration that an actuation cell is deflating) may be sufficiently short in duration such that the inflation and/or deflation is perceived by a user as a localized impulse. In some cases, the duration of the actuation is less than about 0.5 seconds. In some cases, the duration of the actuation is less than about one second. In some cases, the duration of the actuation is less than about five seconds.

In some cases, the duration of an actuation may be relatively long (e.g., greater than about five seconds, greater than about 10 seconds). Similarly, a static period (e.g., a duration that an actuation cell 201 is not inflating or deflating) may be relatively short (e.g., less than about 0.5 seconds, less than about one second, less than about five seconds) or relatively long (greater than about five seconds, greater than about 10 seconds). The lengths of inflation periods, deflation periods, and static periods may be varied to provide varying haptic outputs or portions of haptic outputs. For example, a relatively short inflation period, deflation period, and/or static period may be perceived as a higher-energy pulse or a tap, while a relatively long inflation period, deflation period, and/or static period may be perceived as a lower-energy output.

In some cases, the haptic outputs include localized haptic outputs produced by one or more actuation cells 201, in which a portion of the top external surface 216 is locally displaced (e.g., moved) and/or deformed (e.g., changed in shape) relative to other portions of the top external surface. Localized haptic outputs may simulate a pulse or a tap. In some cases, the haptic outputs include global haptic outputs in which many actuation cells 201 cooperate to displace and/or deform all or substantially all (e.g., greater than 75%) of the top external surface.

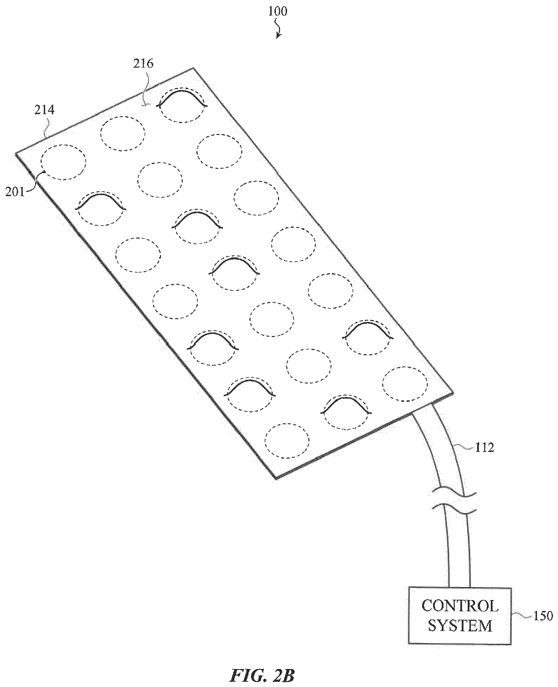

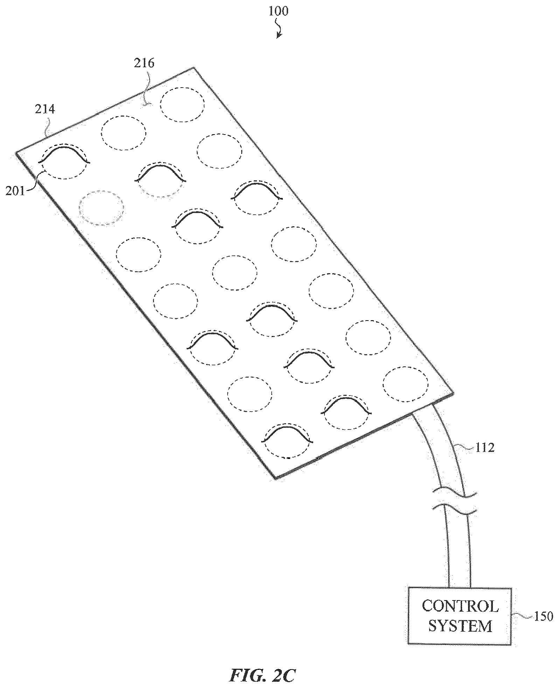

In some cases, multiple actuation cells 201 may cooperate to produce a haptic output. Multiple portions of the top external surface 216 may be displaced and/or deformed by actuation of multiple different actuation cells 201 to produce a haptic output. In some cases, multiple different portions of the top external surface 216 are displaced and/or deformed in different manners according to a pattern to provide a haptic output. In some cases, actuation of the actuation cells 201 in a predetermined sequence may cause the external surface 216 to displace and/or deform according to an actual or simulated randomized pattern (e.g., no ordered pattern is discernable). In some cases, for example, the actual or simulated randomized pattern may simulate a pattern of falling raindrops. For example, a first group of one or more actuation cells 201 may inflate (partially or fully) at a first time or part of a predetermined sequence as shown in FIG. 2B, and a second group of one or more actuation cells 201 may inflate (partially or fully) at a second time or part of a predetermined sequence as shown in FIG. 2C. Different actuation cells 201 of the array of actuation cells may have inflation periods, deflation periods, and/or static periods having different lengths during a haptic output. Similarly, the same actuation cell 201 may have inflation periods, deflation periods, and or static periods having different lengths during a haptic output. In some cases, the inflation periods, deflation periods, and/or static periods of an actuation cell 201 may overlap in time with the inflation periods, deflation periods, and/or static periods of other actuation cells during a haptic output.

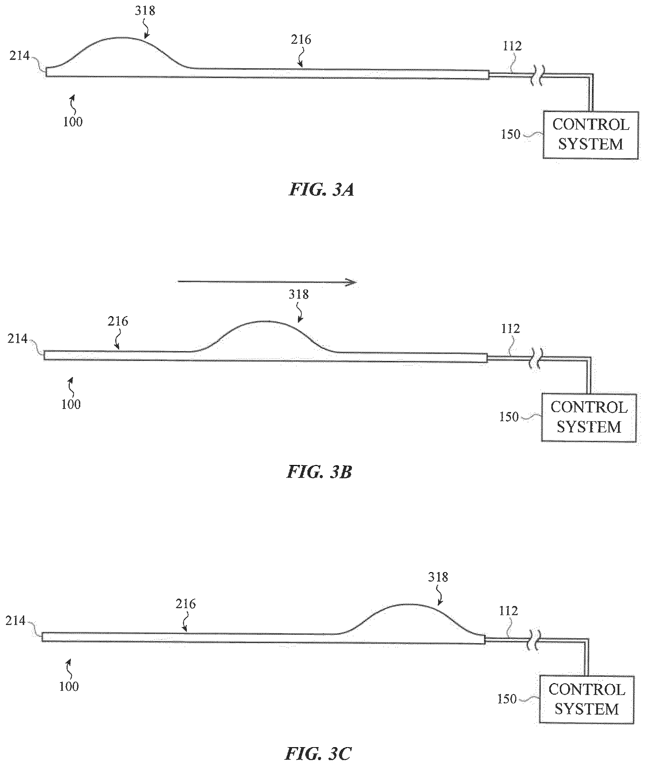

In some cases, actuation of the actuation cells 201 in a predetermined sequence may cause the external surface 216 to displace and/or deform according to an ordered (e.g., non-random) pattern. Multiple actuation cells 201 may cooperate to displace and/or deform the external surface 216 according to an ordered pattern. For example, as shown in FIGS. 3A-3C, the ordered pattern may cause a feature 318 formed by one or more actuation cells 201 to move along the external surface 216. In some cases, for example, the ordered pattern may simulate a wave or other feature moving at least partially across the external surface 216 of the in-bed haptic device 100.

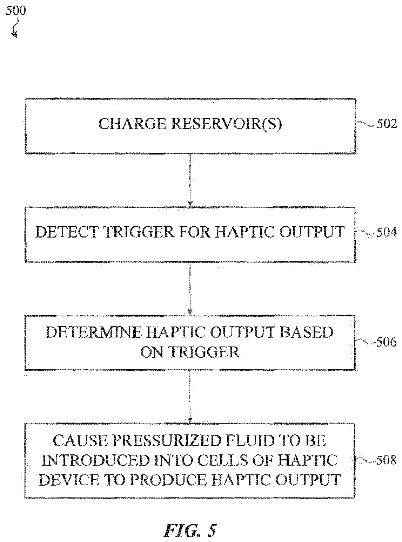

The in-bed haptic device 100, the control system 150, and/or another device that includes and/or is operably connected to a processing unit may include one or more input devices (e.g., contact sensors, force sensors, audio sensors, biometric sensors, images sensors, light sensors, and the like) configured to detect inputs that are used by the processing unit to determine to provide haptic outputs and/or the types of haptic outputs to provide. The processing unit may determine a haptic output to provide in response to one or more detected inputs, and may control various components of the control system 150 and/or the in-bed haptic device 100 (e.g., valves, pumps, etc.) to provide the haptic output. Input devices and detected inputs are discussed in more detail below with respect to FIGS. 4 and 5.

As noted above, the in-bed haptic device may be fluidly coupled to a control system that is configured to introduce pressurized air into the actuation cells and/or remove pressurized air from the actuation cells. FIG. 4 shows an example block diagram of a control system 450 that is fluidly coupled to an in-bed haptic device 400. The control system 450 and the in-bed haptic device 400 may be similar to the control system 150 and the in-bed haptic device described above, and may include similar structure and/or functionality.

The control system 450 may include a high pressure reservoir 454, a vacuum reservoir 456, pumps 458a, 458b, a valve array 452, and a processing unit 460. The in-bed haptic device may include an array of actuation cells 401a, 401b, 401c, 401d that are configured to actuate (e.g., expand, contract, or otherwise change shape) in a predetermined sequence to provide haptic outputs. In some cases, each actuation cell 401 includes one or more bladders configured to inflate and/or deflate to actuate the actuation cells.

The control system 450 may include one or more reservoirs 454, 456 configured to facilitate rapid inflation and/or deflation of bladders of the actuation cells 401. In some cases, the control system 450 includes one or more high pressure reservoirs 454 containing air (or another fluid) having a pressure that is higher than atmospheric pressure and/or one or more vacuum reservoirs 456 containing air (or another fluid) having a pressure that is lower than atmospheric pressure. The reservoirs 454, 456 may be any suitable containers having a fixed or variable volume, such as a tank, a bladder, or the like. The reservoirs 454, 456 may be formed of any suitable material(s), including polymers (e.g., PVC, polyurethane, NOMEX, HYPALON, thermoplastic, polyethylene, polyimide, cellulose, etc.), rubber, synthetic rubber, metal (e.g., aluminum, copper, etc.), fiber reinforced materials, composite materials, and the like.

The control system 450 may include one or more pumps 458a, 458b configured to establish and maintain the pressure(s) of the reservoirs 454, 456. The control system 450 may include a pressurizing pump 458a configured to increase the pressure and/or maintain the increased pressure in the high pressure reservoir 454. The control system may include a vacuum pump 458b configured to decrease the pressure and/or maintain the decreased pressure in the vacuum reservoir 456.

Using the reservoirs 454, 456 for inflating and/or deflating the bladders of individual actuation cells 401 of the in-bed haptic device 400 may allow the individual cells to be inflated and/or deflated more rapidly than using pumps to inflate and/or deflate the bladders. The pumps 458a, 458b of the control system 450 can pressurize or depressurize the reservoirs 454, 456 over a long period of time in advance of providing haptic outputs to "charge" the reservoirs so that more rapid pressure changes may occur. In addition, using the reservoirs 454, 456 for inflating and/or deflating the bladders of the in-bed haptic device 400 may reduce potential disturbances (e.g., sound, vibration, and the like) that would be created by using a pump for inflation and/or deflation. For example, the pumps 458a, 458b of the control system 450 can pressurize or depressurize the reservoirs 454, 456 before use of the in-bed haptic device 400 (e.g., while a user is not present), thereby minimizing disturbances to users. The pumps 458a, 458b may be any suitable type of pump or compressor, including diaphragm-type, piston- or reciprocating-type, plunger-type, rotary-type, scroll-type, diffusion-type, sublimation-type, sorption-type, ion-type, and the like. In some cases, the pumps 458a, 458b are combined in a single compressor/vacuum pump that is capable of pressurizing and depressurizing the reservoirs 454, 456. The pumps 458a, 458b may be sufficiently quiet that they do not disturb users while sleeping. For example, the pumps 458a, 458b may include scroll-type, or rotary-type, or diffusion-type compressors or pumps.

The in-bed haptic device 400 may be fluidly coupled to the control system 450 by one or more connectors 412a, 412b, 412c, 412d. In some cases, each connector 412a, 412b, 412c, 412d connects one or more reservoirs 454, 456 of the control system 450 to a respective actuation cell 401a, 401b, 401c, 401d. As noted above, each actuation cell 401 may be individually addressed. To facilitate the independent control of the actuation cells 401, each actuation cell may be independently fluidly coupled (or capable of being fluidly coupled) to the control system 450. For example, each actuation cell 401 may be fluidly coupled to the control system 450 by one or more fluid paths defined by the in-bed haptic device 400, the connectors 412a, 412b, 412c, 412d, and/or the control system 450. Each of connectors 412a, 412b, 412c, and 412d may define at least a portion of a fluid path between the reservoirs 454, 456 and the bladders of a respective actuation cell 401a, 401b, 401c, 401d.

In some cases, the connectors 412a, 412b, 412c, and 412d cooperate with passages defined in the in-bed haptic device 400 and/or the control system 450 to fluidly coupled one or more reservoirs 454, 456 of the control system 450 to a respective actuation cell 401a, 401b, 401c, 401d. The connectors 412 of the control system 450 allow the control system 450 and the in-bed haptic device 400 to be positioned separately from one another. In some cases, the control system 450 may be located far enough away from the in-bed haptic device 400 (and the user), such as in another room, that potential disturbances (e.g., sounds, vibrations, and the like) produced by the control system may not disturb a user.

The connectors 412a, 412b, 412c, 412d may be separate from one another (e.g., hoses, tubes, etc.) or may be passages through one or more shared components. The connectors 412a, 412b, 412c, 412d may be formed of any suitable material(s), including polymers (e.g., PVC, polyurethane, NOMEX, HYPALON, thermoplastic, polyethylene, polyimide, cellulose, etc.), rubber, synthetic rubber, metal (e.g., aluminum, copper, etc.), fiber reinforced materials, composite materials, and the like.

The control system 450 may include one or more valves (e.g., a valve array 452) configured to control the fluid coupling between each actuation cell 401a, 401b, 401c, 401d (e.g., the bladder(s) of each actuation cell 401) and the one or more reservoirs 454, 456. A valve of the valve array 452 may be opened to fluidly couple one or more bladders to a reservoir 454, 456 via one or more connectors so that fluid may flow between the bladders and the reservoir. Similarly, a valve of the valve array 452 may be closed to terminate a fluid coupling so that fluid may not flow between the bladders and the reservoir 454, 456. The processing unit 460 of the control system 450 may cause a valve between a bladder of an actuation cell 401 and the high pressure reservoir 454 to open to inflate the bladder. Similarly, the control system 450 may cause a valve between a bladder of an actuation cell 401 and the vacuum reservoir 456 to deflate the bladder. In some cases, the valves may be used to modulate the flow between actuation cells 401 and a reservoir 454, 456. For example, a flow may be decreased or increased using a valve.

The valves (e.g., the valve array 452) may be positioned at any suitable location along the fluid path between a reservoir 454, 456 and one or more bladders, including within the control system 450, connector(s) 412, in-bed haptic device 400, or actuation cells 401. The valves of the valve array 452 may be operably coupled to the processing unit 460 by a connector 464c. The valve array may include one or more motors, servos, or the like to control (e.g., open, close) the valves in response to signals received from the processing unit 460. The valves of the valve array 452 may be any suitable type of valves, including ball valves, butterfly valves, choke valves, diaphragm or membrane valves, gate valves, globe valves, knife valves, needle valves, pinch valves, piston valves, plug wave valves, solenoid valves, spool valves, or the like.

The in-bed haptic device 400 may be operably coupled to the processing unit 460 by a connector 464d. The processing unit 460 may receive signals (e.g., sensor signals, etc.) from the in-bed haptic device 400 and provide signals (e.g., valve control signals, etc.) to the in-bed haptic device. In some cases, the processing unit 460 determines to provide a haptic output and/or a type of haptic output to provide in response to signals received from the in-bed haptic device 400. The processing unit 460 may be positioned within and/or be a component of the in-bed haptic device 400, the control system 450, or another device (e.g., a companion device).

The in-bed haptic device 400, the control system 450, and/or another device that includes and/or is operably connected to a processing unit 460 may include one or more input devices (e.g., contact sensors, force sensors, audio sensors, biometric sensors, images sensors, light sensors, and the like) configured to detect inputs that are used by the processing unit to determine to provide haptic outputs and/or the types of haptic outputs to provide. The processing unit 460 may determine a haptic output to provide in response to one or more detected inputs, and may control various components of the control system 450 and/or the in-bed haptic device 400 (e.g., valves, pumps, etc.) to provide the haptic output.

In some cases, the input devices include one or more force sensing mechanisms for detecting input signals for use in providing haptic outputs. The force sensing mechanisms may be capable of detecting whether a user is in bed, a positioning of the user in bed, heart information, breathing information, and the like. The force sensing mechanisms may include capacitive sensing mechanisms, piezoelectric sensing mechanisms, and the like. In some cases, the input devices include one or more contact sensing mechanisms (e.g., touch and/or proximity sensing mechanisms) for detecting input signals for use in providing haptic outputs. The contact sensing mechanism may be capable of detecting whether a user is in bed, for example by detecting that the user is contacting the bed and/or the in-bed haptic device 400. The contact sensing mechanism may additionally be capable of detecting a positioning of the user in bed, (e.g., whether the user is sleeping on his or her back, side, or stomach, a relative positioning of the user in the bed, or the like). The contact sensing mechanisms and/or force sensing mechanisms may use mutual-capacitive sensing techniques and/or self-capacitive sensing techniques. The contact sensing mechanisms and/or force sensing mechanisms may include a substrate and capacitive, piezoelectric and/or other sensing mechanisms that include one or more electrodes for determining whether a user is in contact with, proximate to, and/or exerting a force on the in-bed haptic device 400 or another device.

In some cases, the input devices include a microphone for detecting audio inputs. In some cases, the audio inputs may be used to detect snoring or other audio data as the in-bed haptic device 400 is used.

The inputs received by the processing unit 460 may be used to determine triggers for providing haptic outputs. Triggers may indicate that a haptic output is to be produced and/or characteristics of the haptic output, and are discussed in more detail with respect to FIG. 5. In response to detecting or determining one or more triggers, the processing unit 460 may determine one or more haptic outputs to be provided and cause the control system 450 and/or the in-bed haptic device 400 to provide the haptic output(s).

FIG. 5 shows a flowchart of an example method 500 for providing a haptic output using an in-bed haptic device. At block 502, a pump (e.g., pumps 458a, 458b) charges (e.g., pressurizes or depressurizes) air or another fluid within one or more reservoirs (e.g., reservoirs 454, 456). For example, as discussed above, in some cases, a control system (e.g., control system 450) includes a high pressure reservoir containing pressurized air and a vacuum reservoir containing depressurized air. In some cases, a processing unit (e.g., processing unit 460) causes the pump to charge the reservoirs. As noted above, charging the reservoirs may allow more rapid and more frequent inflation and/or deflation of actuation cells used to provide haptic outputs.

At block 504, the processing unit detects a trigger indicating that a haptic output should be produced using the in-bed haptic device. Triggers may include user conditions that indicate whether a user is asleep or awake, present or not present, snoring or not snoring, and the like. User conditions may be determined by analyzing signals from input devices (e.g., contact sensors, force sensors, audio sensors, biometric sensors, images sensors, light sensors, and the like). For example, the processing unit may determine user conditions by determining breathing information (e.g., instantaneous breathing rate, average breathing rate, maximum breathing rate, minimum breathing rate), user movement or presence information, heart information (e.g., instantaneous heart rate, average heart rate, maximum heart rate, minimum heart rate) determined from contact sensors, force sensors, audio sensors, biometric sensors, images sensors, light sensors, and the like. Triggers may also include raw inputs received from the in-bed haptic device and/or other devices, outputs provided by a device (e.g., audio outputs, video outputs, haptic outputs, alerts or alarms, and the like), and other conditions (e.g., time of day, temperature, humidity, weather, and other environmental conditions). In some cases, the processing unit may determine whether a received input or determined trigger exceeds a threshold level. In some cases, the method only proceeds if the input or trigger exceeds the threshold level.

At block 506, the processing unit determines a haptic output to produce. In response to detecting or determining one or more triggers, the processing unit may determine one or more haptic outputs to be provided. For example, the processing unit may determine a pattern, predetermined sequence, or the like associated with the haptic output. In some cases, haptic output is determined in response to detecting the trigger at block 504. In some cases, the haptic output corresponds to one or more characteristics of an input detected by an input device.

In some cases, determining the haptic output may include determining an amount of haptic feedback to be produced (e.g., a magnitude of the haptic output, a length of haptic output, or the like). The processing unit may determine an amount of the haptic feedback to be produced based, at least in part, on a characteristic of an input detected by the input device.

At block 508, the processing unit causes pressurized fluid (e.g., air) to be introduced into one or more actuation cells of the in-bed haptic device to produce the haptic output. As noted above, the pressurized air may be introduced into the one or more actuation cells according to a predetermined sequence. In some cases, providing the haptic output include deflating one or more actuation cells (e.g., removing the fluid from the actuation cells). For example, an actuation cell may inflate (partially or fully) to provide a first portion of a haptic output and may deflate (partially or fully) to provide a second portion of a haptic output. In addition, an actuation cell may remain static (e.g., deflated, partially inflated, or fully inflated) during a haptic output (e.g., between inflation or deflation or while one or more other actuation cells inflate or deflate).

As noted above, causing the pressurized fluid to be introduced into and/or removed from the actuation cell(s) may include opening and/or closing one or more valves of a valve array to control a fluid coupling between a reservoir of the control system and one or more bladders of an actuation cell. The processing unit of the control system may cause a valve between a bladder of an actuation cell and the high pressure reservoir to open to inflate the bladder. Similarly, the control system may cause a valve between a bladder of an actuation cell and the vacuum reservoir to deflate the bladder.

Using the reservoirs for inflating and/or deflating the bladders of individual actuation cells of the in-bed haptic device may allow the individual cells to be inflated and/or deflated more rapidly than using pumps to inflate and/or deflate the bladders. The pumps of the control system 450 can pressurize or depressurize the reservoirs over a long period of time in advance of providing haptic outputs to "charge" the reservoirs so that more rapid pressure changes may occur. In addition, using the reservoirs for inflating and/or deflating the bladders of the in-bed haptic device may reduce potential disturbances (e.g., sound, vibration, and the like) that would be created by using a pump for inflation and/or deflation. For example, the pumps of the control system can pressurize or depressurize the reservoirs before use of the in-bed haptic device (e.g., while a user is not present), thereby minimizing disturbances to users.

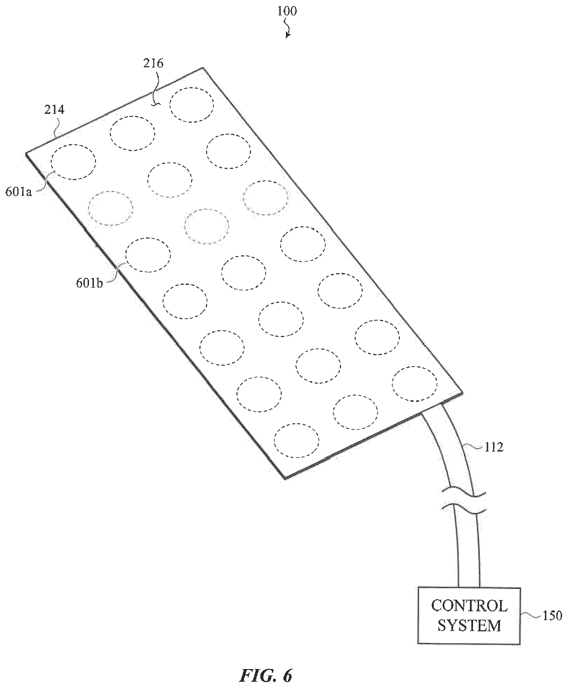

FIG. 6 shows a sensor array embodiment of the in-bed haptic device 100 and control system 150 of FIG. 1. As noted above, the in-bed haptic device 100 can include an array of sensing devices 601 (shown in phantom in FIG. 6) that are configured to sense one or more parameters of a user positioned over the in-bed haptic device 100. In some cases, each sensing device 601 can be configured to sense a pressure and send a signal indicative of the sensed pressure to the control system 150. The control system 150 can use pressure measurements and a location of each sensing device 601 to generate a pressure map. For example, the control system 150 can associate pressure measurements received from a first pressure sensing device 601a with a location of the first pressure sensing device 601a along the enclosure 214. Similarly, the control system 150 can associate pressure measurements from a second pressure sensing device 601b with a location of the second pressure sensing device 601b along the enclosure 214. In this regard, the control system 150 can aggregate pressure measurement from pressure sensing devices 601 to generate a pressure map for a user positioned on the in-bed haptic device 100.

In some cases, each sensing device 601 can be implemented as an actuation cell. For example, each sensing device 601 can include a pressure sensor that is configured to sense a fluid pressure inside a corresponding actuation cell. The control system 150 can monitor and adjust a fluid pressure within each actuation cell while performing measurements. For example, prior to a user lying or sitting on the haptic device 100, the control system 150 can pressurize each actuation cell to a defined pressure, and once a user lies or sits on the haptic device 100, the control system 150 can be configured to determine changes in pressure in each actuation cell based on measurements from a corresponding pressure sensor. In other cases, the controller 150 can be configured to adjust a pressure in the array of pressure sensing devices 601 during a pressure measurement. In a first set of examples, the actuation cells of the pressure sensing devices 601 can be pressurized to a first pressure and the controller can be configured to provide pressurized air to the actuation cells of the pressure sensing device 601 and measure pressure changes and/or volume changes in each pressure sensing device 601. The pressure-volume response of the pressure sensing device 601 can be used to generate a pressure map for a user positioned on the haptic device 100. In a second set of examples, the pressure sensing device 601 can be adjusted while a user is lying on or otherwise positioned on the haptic device 100. This can be used to increase the dynamic sensing range of the sensing devices 601. For example, if the pressure exerted by a user on the haptic device 100 completely compresses one or more of the actuation cells, the controller 150 can be configured to increase a pressure in the actuation cell to re-inflate the cell such that the pressure sensor can continue to measure pressure changes. As described herein, the control system 150 can be configured to independently control pressure and/or volume in each actuation cell. Accordingly, in embodiments where each sensing device 601 includes an actuation cell, the control system 150 can independently measure and control pressures within each auction cell of the array of pressure sensing devices 601.

The array of pressure sensing devices 601 can be implemented in a variety of different ways. In some cases, the haptic device 100 can include just an array of sensing devices 601 and may not include actuation cells. In other cases, the haptic device 100 can include an array of sensing device 601 that are independent from the array of actuation cells. For example, the in-bed haptic device 100 can include two arrays, a first array of actuation cells as described herein and a second array of sensing devices 601. In these cases, the actuation cells and sensing devices 601 may be positioned in different configurations such as an alternating arrangement, overlapping, or any other suitable arrangement. In this regard, the actuation cells and sensing devices 601 may be independent in that the sensing devices 601 directly measure the pressure of a user on the haptic device 100 instead of measuring fluid pressures or changes in fluid pressures within each actuation cell.

Additionally or alternatively, the in-bed haptic device 100 can include other sensors that are used to generate array data to determine a profile, position, or posture of a user. The other sensors can be configured in an array as described herein and include temperature sensors, proximity sensors, acoustic sensors, or other types of sensors. For example, acoustic sensors can include a microphone that is used to detect sounds of the user, such as a heartbeat or respiration. Relative sound levels can be used to determine a posture of a user, such as whether he or she is on their back, side, or in some other position. In other examples, the array of sensors can include one or more motors that are used to vibrate the haptic device 100, and the sensors can measure changes in resonant frequencies of the vibrations, which can be used to determine a position, posture, or other parameters of a user. In yet other cases, proximity sensors, such as an antenna system, can be used to determine height changes across the array of sensors, which can be used to determine posture, position, a curvature of the user's torso, or other physiological parameters of the user. The haptic device 100 can include a combination of various sensors described herein and the outputs from the various sensors can be analyzed and/or combined by the control system 150 to generate a pressure and/profile map of a user positioned on the haptic device 100, or other physiological parameters of a user, such as a posture, position, heartbeat, and/or respiration.

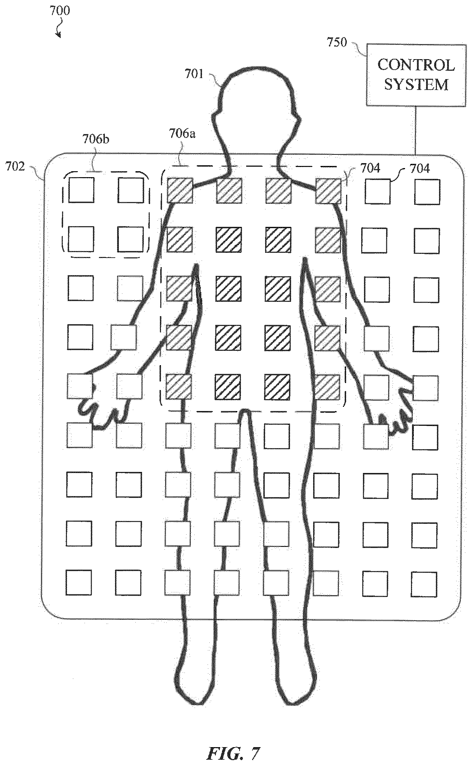

FIG. 7 shows an embodiment of a user positioned on an in-bed haptic device 700. The in-bed haptic device 700 is an example of the haptic devices described herein and includes a sensor array 702 and a control system 750 as described herein. The sensor array 702 can include sensing devices 706 (two of which are labeled for clarity), which can be examples of the sensing devices and/or sensors described herein.

The control system 750 can operate the sensor array 702 to identify one or more subset of sensors 706 that can be used to perform measurements and/or provide feedback to the user 701. For example, the control system 750 can be configured to operate the sensor array to determine a position of the user 701 relative to the sensor array 702. This can include identifying a first subset of sensing devices 706a that are contacted by the user 701 and a second subset of sensing devices 706b that are not contacted by the user 701. In cases where the sensor array 702 includes pressure sensing devices, identifying the first subset of sensing device 706a and the second subset of sensing device 706b can be based on a pressure map determined from outputs of the sensor array 702. Additionally or alternatively, the sensor array 702 can include temperature sensors, proximity sensors, and capacitive sensors, which can be used to identify the first subset of sensing device 706a and the second subset of sensing device 706b.

In some cases, different subsets of sensing devices 706 can be used to provide targeted haptic feedback and/or perform additional sensing functions. For example, the first subset of sensing devices 706a can each include an actuation bladder, and the control system 750 can actuate individual actuation bladders within the first set of sensing device 706a to provide targeted haptic feedback of the user. In some cases, pressure map data can be used to identify a posture and/or position of the user, which can be used to associate a different sensing device 704 with anatomical features of a user. For example, the control system 750 can be configured to determine that the first subset of sensing devices 706a is positioned under a torso section of the user 701. The control system 750 can use this to provide haptic feedback to specific anatomical features, which can include inflating or deflating specific actuation cells.

In some cases, a subset of sensing devices 706a can be used for targeted physiological sensing of a user. For example, in response to determining that the first subset of sensing devices 706 are located under a user, the control system 750 can use one or more sensing devices 704 in the first subset of sensing device 706a to measure a heart rate, respiration rate, or other physiologic parameters of the user 701.In some cases, the array of sensors 702 can include multiple types of sensing devices 704 which can be used for different sensing purposes. For example, the sensing devices 704 can include both pressure sensors such as the actuation cells described herein along with acoustic sensors. In this example, the pressure sensors can be used to generate a pressure map that is used to identify the firs subset of pressure sensing device 706a and the acoustic sensors can be used to measure a physiological parameter of a user which can include a heartbeat, respiration rate, or the like.

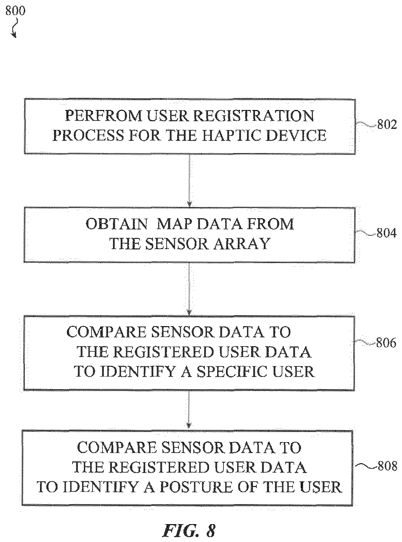

FIG. 8 shows a flowchart of an example method 800 for identifying a user of the haptic device as a specific user from a set of users. The method 800 can be performed by the devices described herein such as the in-bed haptic device.

At step 802, the method 800 includes performing a user registration process for the haptic device. This can include a control system or a companion device such as a smart device instructing a specific user to lie on or otherwise contact a sensor array. The control system can operate the haptic device to generate map data for the user, and associate the map data with the specific user that is performing the registration processes. The map data can include a pressure map, temperature map, proximity data, or other sensor data as described herein. In some cases, the control system and/or companion device can associate multiple different sets of map data with the specific user. For example, at step 802 the user can be instructed to lie in a specific position, which can include instructing a user to lie on their back, side, stomach, or other position. In some cases, multiple measurements can be taken in each position and averaged or otherwise combined to generate a composite map data set for a specific position.

At step 804, the method 800 can include operating the sensor array in a sensing mode that is used to identify a specific user from a set of saved users. The set of saved users can include users that have performed the registration process at step 802. Outputs from the sensing array can be used to generate a map of the user as described herein. In cases where the sensing array includes pressure sensors, the map can be a pressure map. In other examples, the sensor array can include other types of sensors in addition to or alternatively to the pressure sensing devices. For example, the sensing array can include temperature sensors, and a temperature map of the user can be generated. As another example, the sensing array can include proximity sensors, and a profile map of the user can be generated, which can include information about a user's posture and/or position relative to the sensor array.

At step 806, the method 800 can include using the map data (e.g., pressure map, temperature map, etc.) to identify the user as a specific user. For example, the pressure map of the user can be compared to a set of registered pressure maps that is each associated with a specific user. Various methods can be used to match the user's pressure map to a stored pressure map, such as using fitting techniques, regressions, machine learning, image difference comparison, and/or other suitable techniques. The matching can be used to identify a user that is contacting the sensor array.

In addition to or as an alternative to step 806, at step 808, the method 800 can include using the map data to identify a posture of the user. For example, the pressure map of the user can be compared to a set of registered pressure maps that is each associated with different postures. The posture of the user on the sensor array can be determined based on matching the map data of the user to the registered map data that is associated with different postures.

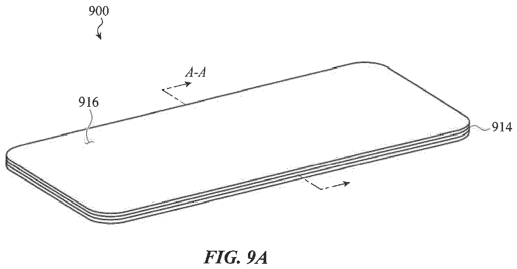

FIG. 9A shows an example in-bed haptic device 900. The in-bed haptic device 900 may be similar to the in-bed haptic devices discussed herein (e.g., in-bed haptic devices 100, 400) and may include similar structure and/or functionality. The in-bed haptic device 900 may include an enclosure 914 that defines a top external surface 916 at which haptic outputs may be provided.

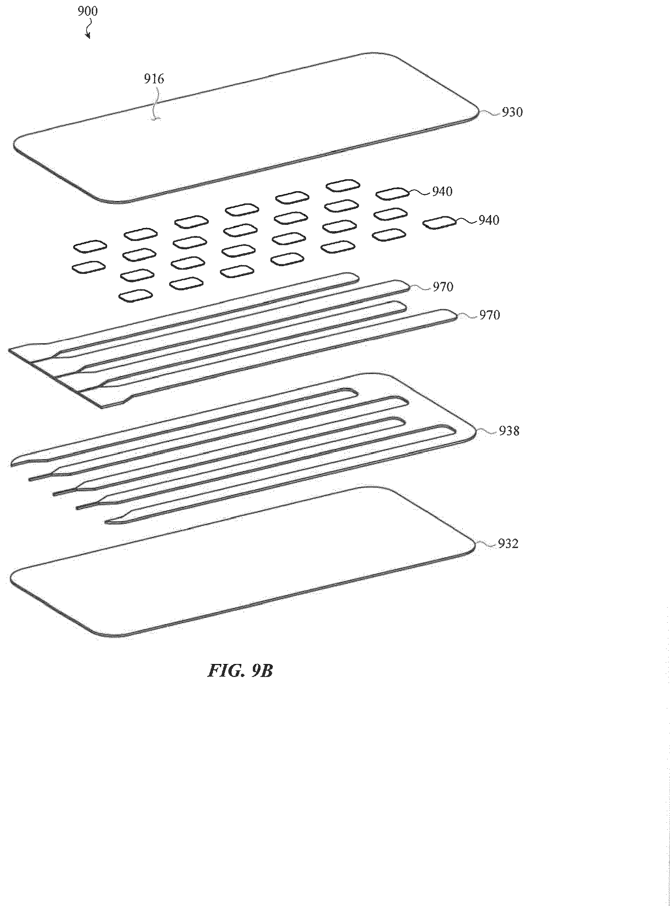

FIG. 9B shows an exploded view of the example in-bed haptic device 900 of FIG. 9A. The in-bed haptic device 900 may include a top layer 930 that defines the top external surface 916 and a bottom layer 932 that defines a bottom external surface of the in-bed haptic device 900. The top layer 930 and the bottom layer 932 may cooperate to define at least a portion of the enclosure 914. The top layer 930 may be formed of any suitable flexible material(s) that is capable of being deformed and/or displaced to provide haptic outputs. In some cases, the top layer 930 is formed of a flexible fabric (e.g., a woven fabric including one or more of nylon, polyester, cotton, or the like). In other cases, the layer 930 may be formed of any suitable material(s), including flexible polymers (e.g., polyurethane, PVC, polyethylene, polyimide, cellulose, etc.), rubbers, synthetic rubbers, fiber reinforced materials, composite materials, and the like.

In some cases, the bottom layer 932 is configured to be positioned above and facing a top surface of a mattress. The bottom layer 932 may be formed of a material or combination of materials that allows the bottom external surface of the in-bed haptic device 900 to adhere or grip a surface upon which it is placed (e.g., a mattress, a bedsheet, a bed, a mattress protector, or another surface). The bottom layer 932 may be formed of a gripping material(s), such as thermoplastic polyurethane. The bottom layer 932 may be formed of any suitable material(s), including fabrics, flexible polymers (e.g., polyurethane, PVC, polyethylene, polyimide, cellulose, etc.), rubbers, synthetic rubbers, fiber reinforced materials, composite materials, and the like. In some cases, the top layer 930 has a stiffness that is less than a stiffness of the bottom layer 932. This may allow the actuation of the actuation cells to deform and/or displace the top layer 930 more than the bottom layer 932 to improve the haptic outputs provided by the in-bed haptic device 900.

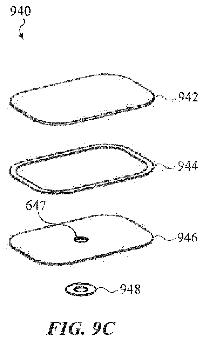

The in-bed haptic device 900 may include an array of actuation cells (e.g., bladders 940) positioned between the top layer 930 and the bottom layer 932. The bladders 940 may be configured to inflate and/or deflate to deform and/or displace the top external surface 916 to provide haptic outputs. The bladders 940 are discussed in more detail below with respect to FIG. 9C.

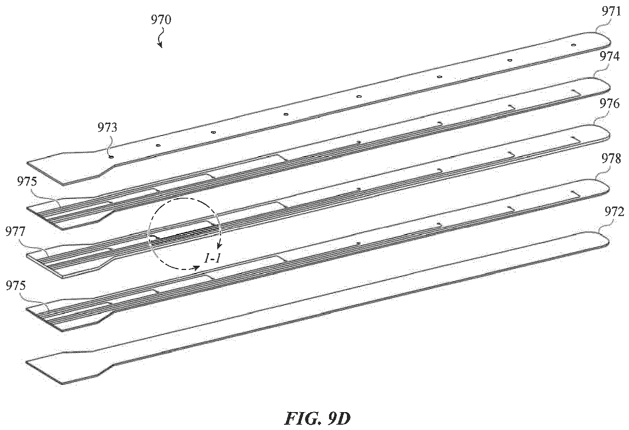

The in-bed haptic device 900 may include passage members 970 positioned beneath the bladders 940. Each passage member 970 may define one or more passages that fluidly couple the bladders 940 to one or more connectors and/or one or more reservoirs of a control system. As shown in FIG. 9B, each passage member 970 may correspond to a row of bladders 940 in the array of actuation cells. Each passage member 970 may define one or more passages that separately fluidly couple each bladder 940 of the row to which the passage member 970 corresponds. The passage members 970 are discussed in more detail below with respect to FIG. 9D.

The in-bed haptic device 900 may include an adhesive layer 938 positioned between the top layer 930 and the bottom layer 932. In some cases, the adhesive layer 938 adheres the top layer 930 to the bottom layer 932 to attach the components of the in-bed haptic device 900 together and form the enclosure 914. In some cases, the adhesive layer 938 and the passage members 970 are positioned in a single layer. For example, the adhesive layer 938 may at least partially surround one or more passage members 970. In some cases, the adhesive layer 938 is positioned in spaces between two or more passage member 970. The adhesive layer 938 may be formed of any suitable type of material(s), including thermoplastic adhesives, pressure-sensitive adhesives, reactive film adhesives, heat-activated films, and the like.

FIG. 9C is an exploded view of an example bladder 940 of FIG. 9B. The bladder 940 may include a top sheet 942, a bottom sheet 946, and an adhesive ring 944 attaching the top sheet 942 and the bottom sheet 946 to define an interior volume. The bottom sheet 946 may include an opening 947 for fluidly coupling the interior volume with a passage of a passage member 970. The bottom sheet 946 may be attached to a passage member 970 using an adhesive ring 948 positioned around the opening 947. The top sheet 942 and bottom sheet 926 may be formed of any suitable material(s), including fabrics, flexible polymers (e.g., polyurethane, PVC, polyethylene, polyimide, cellulose, etc.), rubbers, synthetic rubbers, fiber reinforced materials, composite materials, and the like. The adhesive rings 944 and 948 may be formed of any suitable type of material(s), including thermoplastic adhesives, pressure-sensitive adhesives, reactive film adhesives, heat-activated films, and the like.

FIG. 9D is an exploded view of an example passage member 970 of FIG. 9B. The passage member 970 may include a top sheet 971, a bottom sheet 972, and a channel member 976 positioned between the top sheet and the bottom sheet. The passage member 970 may further include adhesive layers 974 and 978 that attach the top sheet 971 to the channel member 976 and the channel member to the bottom sheet 972, respectively. The channel member 976 may cooperate with the top sheet 971, the bottom sheet 972, and/or the adhesive layers 974, 978 to define passages extending from openings 973 defined in the top sheet 971 and an end of the passage member 970. The openings 973 may fluidly couple the passages to bladders 940 (e.g., via openings 947 of the bladders), and the passages may be used to fluidly couple the bladders 940 to a connector, a reservoir, or the like, for providing haptic outputs using the bladders. The end of the passage member 970 may be connected to a connector to fluidly couple the bladders 940 to a control system.

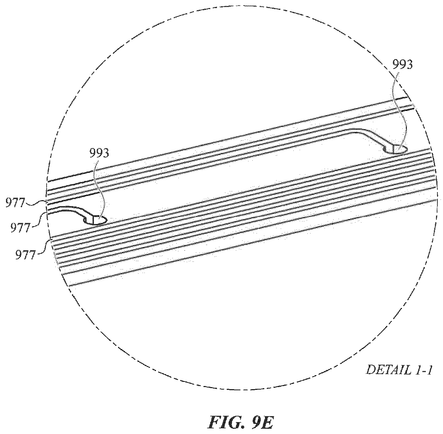

The channel member 976 may include one or more channels 977 extending from an end of the channel member 976 to positions that correspond to the openings 973. FIG. 9E shows a detail view of section 1-1 of FIG. 9D, showing channels 977. The channels 977 may include portions 993 that correspond to the openings 973. Each portion 993 of each channel 977 may align with an opening 973 to fluidly couple one or more bladders 940 to the channel 977. The channels 977 may define sidewalls of each passage, the top sheet 971 may define a top wall of each passage, and the bottom sheet 972 may define a bottom wall of each passage.

In some cases, as shown in FIG. 9D, the adhesive layers 974, 978 may include channels 975 that correspond to the channels 977 of the channel member 976. These channels may prevent the adhesive layers 974, 978 from causing the passages to be blocked. The channel member 976, the top sheet 971 and bottom sheet 972 may be formed of any suitable material(s), including fabrics, flexible polymers (e.g., polyurethane, PVC, polyethylene, polyimide, cellulose, etc.), rubbers, synthetic rubbers, fiber reinforced materials, composite materials, and the like. In some cases, the channel member 976 is formed from thermoplastic polyurethane. The adhesive layers 974, 978 may be formed of any suitable type of material(s), including thermoplastic adhesives, pressure-sensitive adhesives, reactive film adhesives, heat-activated films, and the like.

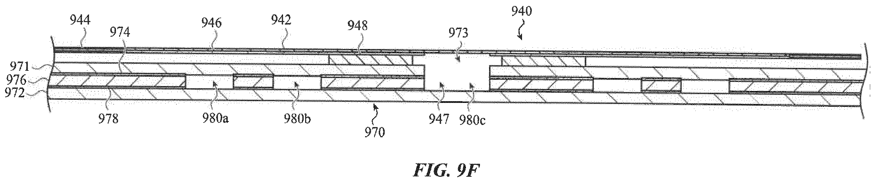

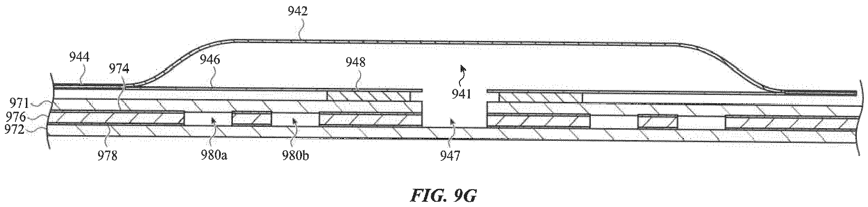

FIG. 9F shows a partial cross-section view of a bladder 940 and a passage member 970, taken through section line A-A of FIG. 9A. As shown in FIG. 9F, the passage member 970 defines passages 980a, 980b, and 980c. The passage 980c fluidly couples the bladder 940 to a control system for providing haptic outputs using the bladder 940. FIG. 9F shows the bladder 940 in a collapsed or uninflated state. FIG. 9G shows the bladder 940 in an inflated state, for example after pressurized air has been introduced to the interior volume 941 of the bladder 940.

In some cases, the channels (e.g., channels 977) may be configured to at least partially control the flow of fluid to one or more bladders. For example, the channels may have a cross-sectional area that decreases along a length of the channel. This may limit the flow of fluid and cause bladders along the length of the in-bed haptic device to inflate at different times and/or rates. For example, a first bladder may be located closer to a first end of the in-bed haptic device, and a first channel fluidly coupled to the bladder may have a first cross-sectional area at the location of the first bladder. A second bladder may be located farther from the first end of the in-bed haptic device, and a second channel fluidly coupled to the bladder may have a second cross-sectional area less than the first cross-sectional area at the location of the second bladder. The smaller cross-sectional area of the second channel at the location of the second bladder may cause the second bladder to inflate at a slower rate and/or at a later time than the first bladder. This may be used to provide a haptic output that varies depending on the location along the surface of the in-bed haptic device, such as the wave described with respect to FIGS. 3A-3C.

In some cases, each bladder may be coupled to two (or more) channels. A cross-sectional area of a first channel fluidly coupled to a bladder may increase in a first direction along the length of the channel, and a cross-sectional area of a second channel fluidly coupled to the bladder may decrease in the first direction. In this manner, the wave or similar haptic output provided using the bladders may be provided in a bi-directional manner depending on which channels are used to introduce fluid to the bladders.

In some cases, multiple channels may be fluidly coupled to a single bladder or multiple bladders. In some cases, a first set of bladders associated with a first waveform of haptic output may be connected to a first common channel, and a second set of bladders associated with a second waveform of haptic output may be connected to a second common channel. In this manner, the first waveform and/or the second waveform may be provided based on whether fluid is introduced into the first common channel and/or the second common channel without the need for complex valve control to individually control each bladder in the set.

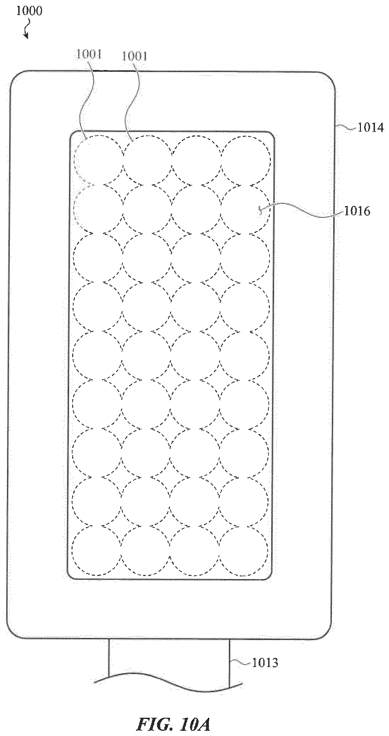

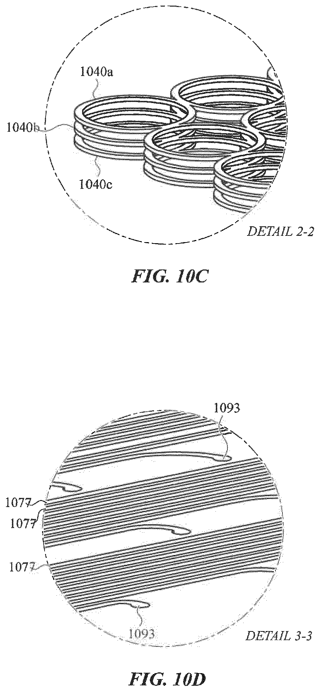

FIG. 10A shows an example in-bed haptic device 1000. The in-bed haptic device 1000 may be similar to the in-bed haptic devices discussed herein (e.g., in-bed haptic devices 100, 400, 900) and may include similar structure and/or functionality. The in-bed haptic device 1000 may include an enclosure 1014 that defines a top external surface 1016 at which haptic outputs may be provided. The in-bed haptic device 1000 may include an array of actuation cells 1001 (shown in phantom in FIG. 10A) positioned beneath the top external surface 1016. As described herein, the actuation cells 1001 may be configured to inflate and/or deflate to deform and/or displace the top external surface 1016 to provide haptic outputs. The in-bed haptic device 1000 may include a connection interface 1013 that fluidly couples the actuation cells 1001 to one or more additional connectors and/or a control system, such as described herein.

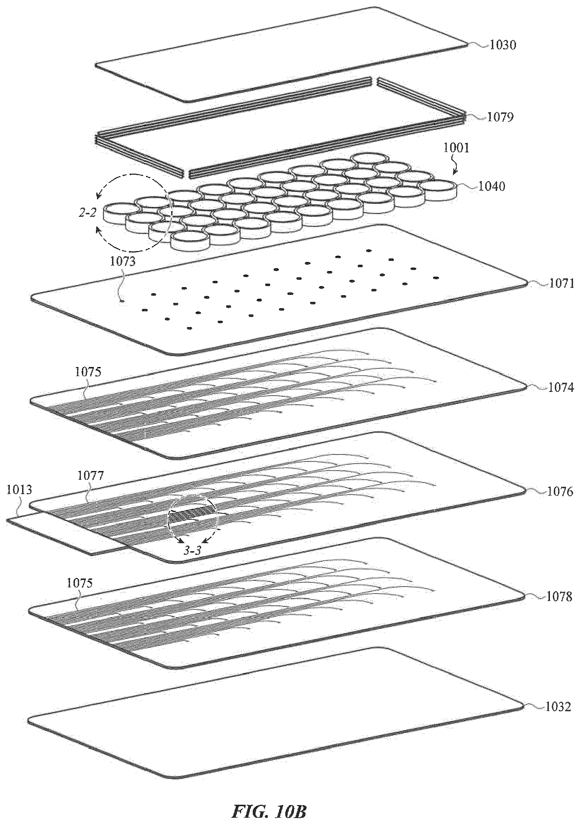

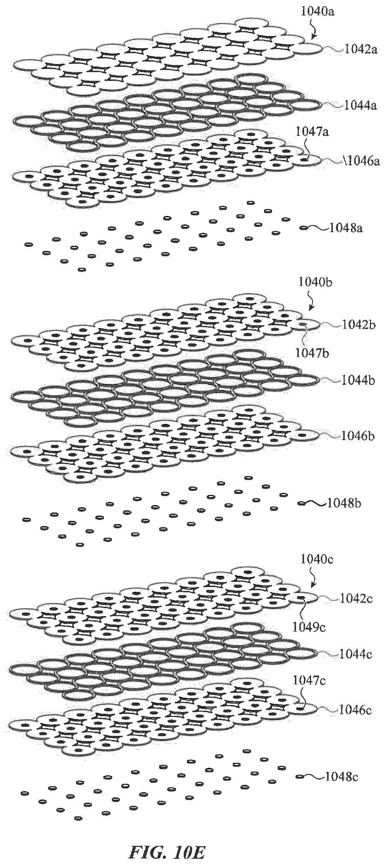

FIG. 10B shows an exploded view of the example in-bed haptic device 1000 of FIG. 10A. The in-bed haptic device may include an array of bladders 1040 that make up the actuation cells 1001. In some cases, each actuation cell 1001 includes multiple bladders 1040 in a stack. For example, three bladders 1040 may be stacked to form an actuation cell 1001. FIG. 10C shows a detail view of section 2-2 of FIG. 10B, showing three bladders 1040a, 1040b, 1040c stacked on top of one another. Including multiple bladders in each actuation cell 1001 may increase an amount that the actuation cell can deform and/or displace the top surface 1016. The bladders 1040 are discussed in more detail below with respect to FIG. 10E.

The in-bed haptic device 1000 may include a top layer 1030 that defines at least a portion of the top external surface 1016 and a bottom layer 1032 that defines a bottom external surface of the in-bed haptic device 1000. The in-bed haptic device 1000 may further include a secondary layer 1071 that defines a portion of the top external surface 1016. In some cases, the secondary layer has a larger surface area than the top layer 1030, as shown in FIG. 10B. The in-bed haptic device 1000 may include one or more actuation components 1079 that attach the top layer 1030 to the secondary layer 1071. In some cases, the actuation components 1079 are flexible and configured to allow the top layer 1030 to move relative to the secondary layer 1071. In some cases, the actuation components 1079 are configured to at least partially surround the actuation cells 1001 to prevent contaminants from coming into contact with the actuation cells and/or prevent other types of damage.

The top layer 1030, the bottom layer 1032, the secondary layer 1071, and the actuation components 1079 may cooperate to define at least a portion of the enclosure 1014. The top layer 1030 may be formed of any suitable flexible material(s) that is capable of being deformed and/or displaced to provide haptic outputs. In some cases, the top layer 1030 is formed of a flexible fabric (e.g., a woven fabric including one or more of nylon, polyester, cotton, or the like). In other cases, the top layer 1030 may be formed of any suitable material(s), including fabrics, flexible polymers (e.g., polyurethane, PVC, polyethylene, polyimide, cellulose, etc.), rubbers, synthetic rubbers, fiber reinforced materials, composite materials, and the like. The bottom layer 1032 may be formed of a material or combination of materials that allows the bottom external surface of the in-bed haptic device 1000 to adhere or grip a surface upon which it is placed (e.g., a mattress, a bedsheet, a bed, a mattress protector, or another surface). The bottom layer 1032 may be formed of a gripping material(s), such as thermoplastic polyurethane. The bottom layer 1032, the secondary layer 1071, and the actuation components 1079 may be formed of any suitable material(s), including fabrics, flexible polymers (e.g., polyurethane, PVC, polyethylene, polyimide, cellulose, etc.), rubbers, synthetic rubbers, fiber reinforced materials, composite materials, and the like.

The in-bed haptic device 1000 may include a channel layer 1076 positioned between the secondary layer 1071 and the bottom layer 1032. The in-bed haptic device 1000 may further include adhesive layers 1074 and 1078 that attach the secondary sheet 1071 to the channel layer 1076 and the channel layer to the bottom sheet 1072, respectively. The channel layer 1076 may cooperate with the secondary sheet 1071, the bottom sheet 1072, and/or the adhesive layers 1074, 1078 to define passages extending from openings 1073 defined in the secondary sheet 1071 and the connection interface 1013. The openings 1073 may fluidly couple the passages to the actuation cells 1001 (e.g., bladders 1040 of the actuation cells), and the passages may be used to fluidly couple the actuation cells to a connector, a reservoir, or the like, for providing haptic outputs using the bladders.

The channel layer 1076 may include one or more channels 1077 extending from an end of the channel layer 1076 (e.g., part of connection interface 1013) to positions that correspond to the openings 1073. FIG. 10C shows a detail view of section 2-2 of FIG. 10B, showing channels 1077. The channels 1077 may include portions 1093 that correspond to the openings 1073. Each portion 1093 of each channel 1077 may align with an opening 1073 to fluidly couple an actuation cell 1001 to the channel 1077. In some cases, the channel layer 1076 and one or more additional layers (e.g., the secondary sheet 1071 and the bottom sheet 1072) may form a passage member, such as the passage member 970 discussed above with respect to FIGS. 9A-9G. Sidewalls of the channels 1077 may define sidewalls of each passage, the secondary sheet 1071 may define a top wall of each passage, and the bottom sheet 1072 may define a bottom wall of each passage.

In some cases, as shown in FIG. 10B, the adhesive layers 1074, 1078 may include channels 1075 that correspond to the channels 1077 of the channel layer 1076. These channels may prevent the adhesive layers 1074, 1078 from causing the passages to be blocked. The channel layer 1076, the top may be formed of any suitable material(s), including flexible polymers (e.g., polyurethane, PVC, polyethylene, polyimide, cellulose, etc.), rubbers, synthetic rubbers, fiber reinforced materials, composite materials, and the like. In some cases, the channel layer 1076 is formed from thermoplastic polyurethane. The adhesive layers 1074, 1078 may be formed of any suitable type of material(s), including thermoplastic adhesives, pressure-sensitive adhesives, reactive film adhesives, heat-activated films, and the like.

FIG. 10E illustrates an exploded view of the array of actuation cells 1001 of FIG. 10B. As described with respect to FIG. 10C, each actuation cell 1001 includes three bladders 1040a, 1040b, 1040c stacked on top of one another. Including multiple bladders in each actuation cell 1001 may increase an amount that the actuation cell can deform and/or displace the top surface 1016. Each bladder 1040a, 1040b, 1040c includes a top member 1042a, 1042b, 1042c, a bottom member 1046a, 1046b, 1046c, and an adhesive ring 1044a, 1044b, 1044c attaching the top member to the bottom member. Each bladder may include one or more openings for fluidly coupling the bladder to other bladders in the actuation cell and/or passages of the in-bed haptic device. For example, each bladder 1040a may include an opening 1047a in the bottom member 1046a for fluidly coupling the bladder 1040a to the bladder 1040b of the same actuation cell 1001. Each bladder 1040b may include an opening 1049b in the top member 1042b that is aligned with the opening 1047a for fluidly coupling the bladder 1040b to the bladder 1040a of the same actuation cell 1001. Each bladder 1040b may further include an opening 1047b in the bottom member 1046b for fluidly coupling the bladder 1040b to the bladder 1040c of the same actuation cell 1001. Each bladder 1040c may include an opening 1049c in the top member 1042c that is aligned with the opening 1047b for fluidly coupling the bladder 1040c to the bladder 1040b of the same actuation cell 1001. Each bladder 1040c may further include an opening 1047c in the bottom member 1046c for fluidly coupling the bladder 1040c to a passage in the in-bed haptic device 1000.

Each actuation cell 1001 may further include an adhesive ring 1048a (similar to the adhesive ring 948 discussed with respect to FIG. 7C) positioned between and attaching bladder 1040a and bladder 1040b. The adhesive ring 1048a may be positioned around the opening 1047a and the opening 1049b. Each actuation cell 1001 may further include an adhesive ring 1048b positioned between and attaching bladder 1040b and bladder 1040c. The adhesive ring 1048a may be positioned around the opening 1047b and the opening 1049c. Each actuation cell 1001 may further include adhesive ring 1048c positioned beneath the bladder 1040c and configured to attach the actuation cell to the secondary sheet 10101. The adhesive ring 1048c may be positioned around the opening 1047c and the opening 1073 of the secondary sheet 1071.

As shown in FIG. 10C, the components for multiple actuation cells 1001 may be combined into and/or formed from a single sheet or component of material. For example, the top members 1042a, 1042b, 1042c, the bottom members 1046a, 1046b, 1046c, and the adhesive rings 1044a, 1044b, 1044c for multiple actuation cells 1001 in the array of actuation cells may be formed from a single sheet. This may simplify manufacturing by reducing the overall number of components used to form the in-bed haptic device 1000.

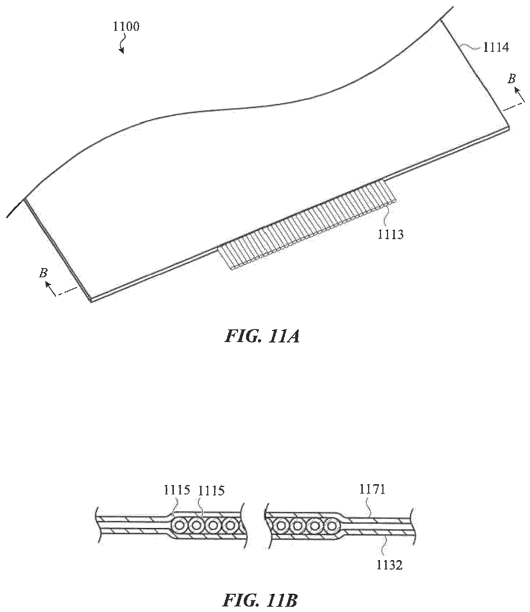

As noted above, the in-bed haptic devices described herein may include a connection interface (e.g., connection interface 1013) for coupling the passages of the in-bed haptic device to connectors and/or a control system. FIGS. 11A-11B illustrate example connection interfaces.

FIG. 11A illustrates an example connection interface 1113 that includes multiple tubular members that are fluidly coupled to individual passages of an in-bed haptic device 1100. The in-bed haptic device 1100 may be similar to the haptic devices described herein, and may include similar structure and/or functionality. FIG. 11B illustrates a cross-section view of the in-bed haptic device 1100, taken through section line B-B of FIG. 11A. As shown in FIG. 11B, the tubular members 1115 are positioned between a first layer 1171 and a second layer 1132 of the haptic device 1100. As shown in FIG. 11A, in some cases, the tubular members 1115 may extend from the enclosure 1114 of the haptic device 1100. The tubular members 1115 may be sealed. For example, the tubular members 1115 may be at least partially encapsulated by a cured adhesive or filler that fills the gaps or voids around the tubular members.

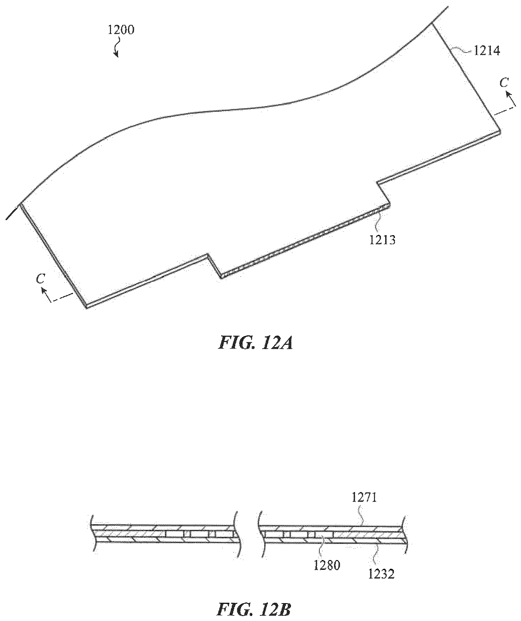

FIG. 12A illustrates an example connection interface 1213 in which passages defined in the in-bed haptic device 1200 are extended to form the connection interface 1213. The in-bed haptic device 1200 may be similar to the haptic devices described herein, and may include similar structure and/or functionality. FIG. 12B illustrates a cross-section view of the in-bed haptic device 1200, taken through section line C-C of FIG. 12A. As shown in FIG. 12B, the passages 1280 are positioned between a first layer 1271 and a second layer 1232 of the haptic device 1200.

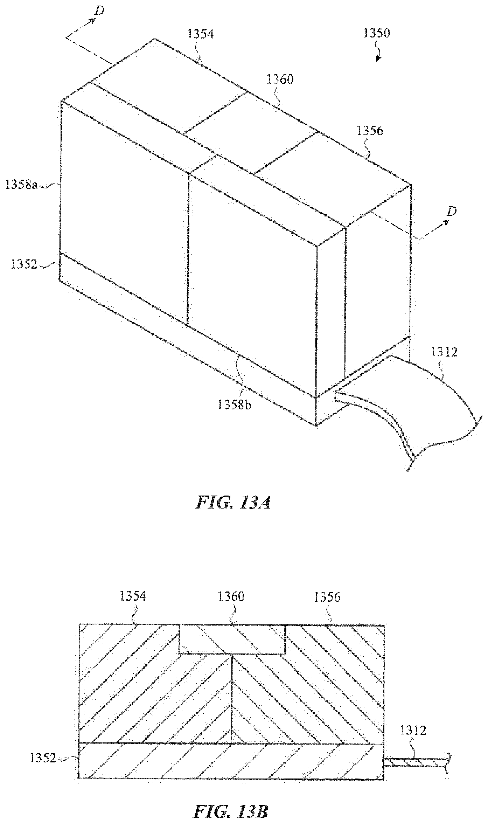

As noted above, the in-bed haptic devices discussed herein may be fluidly coupled to a control system that is configured to introduce pressurized air into the bladders of the array of actuation cells and/or remove pressurized air from the bladders of the array of actuation cells to provide haptic outputs. FIGS. 13A-13B illustrate an example arrangement of example components of a control system module 1350. The control system module 1350 may be similar to the control systems described herein, and may include similar structure and/or functionality. The components of the control system module 1350 may be arranged in a compact package to reduce an overall size of the control system, which may improve a user experience. The control system module 1350 includes pumps 1358a, 1358b, reservoirs 1354, 1356, a valve array 1352, a processing unit 1360, and a connector 1312. FIG. 13B shows a cross-section of the example control system 1350, taken through section line D-D of FIG. 13A. As shown in FIG. 13B, the reservoirs 1354, 1356 may cooperate to define a recess, and the processing unit 1360 may be positioned at least partially within the recess.

In some cases, multiple control system modules 1350 may be combined to form a control system. For example, the control system module 1350 may be stacked or otherwise arranged with one or more additional control system modules to expand the capacity of the system and/or expand the number of actuation cells that can be actuated using the control system.

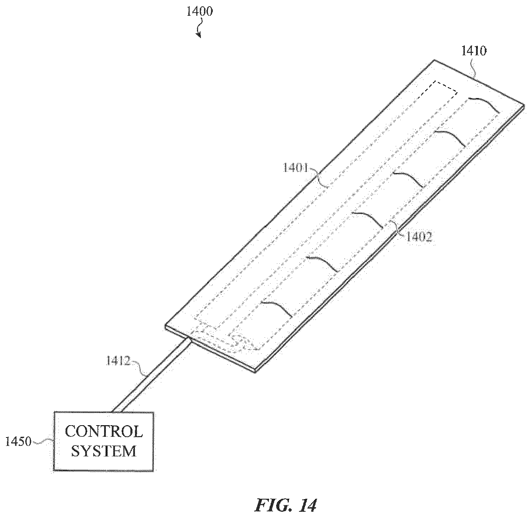

FIG. 14 shows an example of in-bed haptic device 1400 and control system 1450. The in-bed haptic device 1400 can include an electronic sensor strip 1401 and an actuation cell 1402 (shown in phantom in FIG. 14) that are configured to actuate (e.g., expand, contract, or otherwise change shape) to provide haptic outputs. The in-bed haptic device 1400 may include an enclosure 1410 or other external layer that at least partially surrounds the sensor strip 1401 and/or the actuation cells 1402 and/or other components of the in-bed haptic device 1400. The enclosure 1410 may contain and/or protect the sensor strip 1401 and/or the actuation cells 1401 and/or other components of the in-bed haptic device 1400. In some cases, the enclosure 1410 is flexible. One or more surfaces of the enclosure 1410 may include an adhesive or a high-friction material(s) configured to maintain the in-bed haptic device 1400 in place. The in-bed haptic device 1400 can also include one or more connectors 1412 that couple the control system 1450 and/or other system components such as fluid pump(s), valves, reservoirs, or the like to the enclosure 1410.

The sensor strip 1401 can include one or more sensors that are used to measure physiological parameters of a user that is positioned over the strip. In some embodiments, the sensor strip 1401 can be a piezoelectric sensor such as a differential piezo electric sensor that is operative to sense movement, respiration, heartbeat, or other physiological parameters of a user. In some embodiments, the sensor strip 1401 can include one or more temperature sensors that are positioned along the enclosure and are operative to detect a body temperature of a user. In further examples, the sensor strip 1401 can include capacitive sensors, strain sensors, accelerometers, or the like that are used to detect one or more parameters such as weight, position, posture, and/or movement of a user. In some cases, the sensor strip 1401 can include a combination of different types of sensors such as a combination of piezoelectric sensor, temperature sensors, capacitive sensors, strain based sensors, or the like. The sensor strip 1401 can include various sensors that are integrated into a single strip. In other cases the sensor strip 1401 can include multiple discrete sensors positioned at various locations within or on the enclosure 1410.

The actuation cell 1402 can also be integrated with the enclosure 1410. The actuation cell 1402 can include one or more sealed bladders that are configured to expand in response to pressurized air (or another gas or fluid) being introduced into the interior volume of the bladder, and/or deflate in response to pressurized air being removed from the interior volume of the bladder. In some cases, the actuation cell 1402 is configured to expand in a direction that is substantially transverse to the top external surface of a bed, thereby increasing a thickness of a region of the in-bed haptic device 100.

In some embodiments, the actuation cell 1402 can be operated to provide a haptic output to a user as described herein. In other cases, the actuation cell 1402 can be operated to sense one or more parameters of a user. For example, the actuation cell 1402 can be operated to measure presence of the user on the bed, movement of a user, posture or position of a user, number and/or location of different people (or animals) in a bed, physiological parameters such as heart rate, respiratory rate, or the like, or a combination thereof. To operate the actuation cell 1402 to measure one or more parameters of a user, the actuation cell 1402 can be partially inflated to maintain a positive air pressure within the bladder. Changes in air pressure within the bladder can be measured and used to determine the one or more parameters of a user. For example, one or more pressure sensors can be used to measure a fluid pressure with the bladder. In some cases, the pressure sensors can include resistive, capacitive, or other pressure measurement technologies. In some cases, the haptic device 1400 can include multiple actuation cells 1402 and pressures within each cell can be used to determine a parameter of a user. For example, internal pressures between different actuation cells 1402 can be compared to determine a location of a user on a bed. In other cases, the actuation cell 1402 can include multiple bladders that are fluidly coupled by one or more fluid passages. In these cases, the actuation cell 1402 can include multiple pressure sensors located at different ones of the bladders, and pressure measurements from the different sensors can be used to determine a parameter of a user. For example, comparing data from the multiple pressure sensors can be used to identify a pressure pulse traveling across the bladders, which may correspond to a heartbeat or other movements of a user.

The control system 1450 can be an example of the control systems described herein. The control system 1450 can include one or more pumps, reservoirs, valves array, a processing unit, pressure sensors, and the like. The control system 1450 can be coupled to the sensor strip 1401 and the actuation cell 1402 by one or more connectors 1412. In some cases, one or more of the pump(s), reservoir(s), valve(s), pressure sensor(s) and/or processing unit can be integrated into the enclosure 1410.

The connector 1412 can include a hybrid cable that integrates electrical power cables, signal lines, and fluid tubing (e.g., pneumatic or hydraulic tubing) to couple the control system 1450 to the sensor strip 1402 and actuation cells 1402. In some cases, the connector 1412 can include an outer housing that encloses the power cables, signal lines and pneumatic or hydraulic tubing. In some cases, the connector 1412 can include multiple power cables, signal lines and/or fluid tubing.

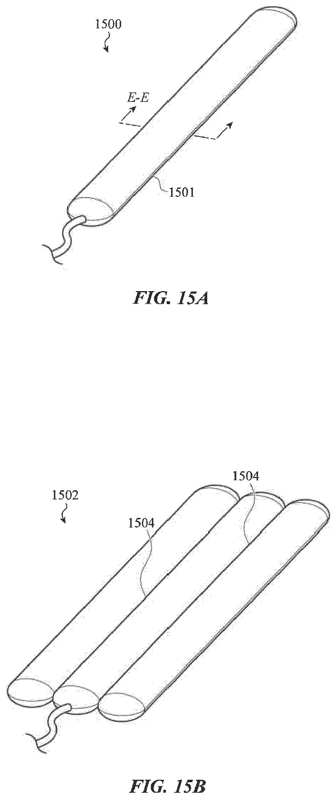

FIG. 15A shows an example of an actuation cell 1500 that can be integrated into the in-bed device as described herein. The actuation cell 1500 can include a bladder that defines a sealed interior volume that is configured to hold a fluid such as air, gas, or liquid. In some cases, the actuation cell 1500 can form an elongated tube structure that is integrated with an in-bed device to extend across a width of sleeping surface such as a bed. The actuation cell 1500 can be formed from a flexible material such as silicone, polyurethane, rubber, synthetic rubber, fiber reinforced materials, composite materials, and the like. When deflated, the actuation cell 1500 can collapse on itself to form a substantially flat structure that does not contain the fluid (or contains little or less fluid). When inflated, the actuation cell 1500 can expand to form a raised structure as described herein. In the example shown in FIG. 15A, the actuation cell 1500 is sealed along its perimeter 1501 to define a closed volume. The actuation cell 1500 can be sized to extend across all or a portion of a user's torso when the user is lying on the in-bed device.

FIG. 15B shows another example of an actuation cell 1502 that can be integrated into the in-bed device as described herein. The actuation cell 1502 can include a bladder that has one or more connections 1504 to form surface features when the bladder is inflated. For example, the connections 1504 can extend along a length of the actuation cell 1502 to form multiple cylindrical features along an exterior surface of the bladder. In some cases, these cylindrical features can extend parallel to each other and parallel to a length dimension of the actuation cell 1502. When inflated, these surface features can apply a different haptic experience to a user by creating different patterns of raised and lower portions that apply varying levels of pressure to a user. FIG. 15B illustrates one example of surface features that can be created, and different portions of the actuation cell 1502 can be connected to form various different surface features when inflated. When deflated, the actuation cell 1502 can collapse on itself to form a substantially flat structure that does not contain the fluid (or contains little or less fluid). In some cases, the connections 1504 are formed by coupling a top portion of the actuation cell to a bottom portion of the actuation cell, which can be accomplished using thermal bonding techniques such as heat sealing, adhesives, mechanical couplers, or any other suitable technique.

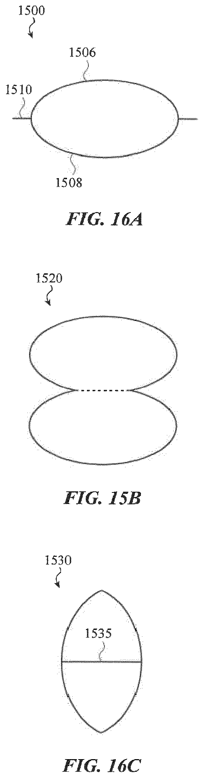

FIG. 16A shows an example cross-sectional view of an actuation cell 1500 taken along line E-E shown in FIG. 15A. As illustrated in FIG. 16A, the actuation cell 1500 can take on a circular or semi-circular configuration when inflated. The actuation cell 1500 can be formed by joining a top section 1506 with a bottom section 1508 along a seam 1510 to create a sealed internal volume. In this regard, the outer profile of the actuation cell 1500 can take on different shapes when inflated based on the shape of the top and bottom sheets 1506 and 1508, and/or the locations where the different sheets are sealed together. For example, FIG. 16B illustrates an alternative example cross-sectional view of an actuation cell 1520. The actuation cell 1520 can include multiple stacked structures, which can be used to increase an expanded height of the actuation cell 1520. The actuation cell 1520 can include multiple circular or semi-circular cross-sectional structures that are joined to form a sealed internal volume. When the actuation cell 1520 is inflated, the height of the actuation cell 1520 can be about two times the diameter of each of the cylindrical sections. FIG. 16C illustrates another example cross-sectional view of an actuation sell 1530 that can be implemented in an in-bed device. In the example of 13C, the actuation cell 1530 can include an internal structure 1535 that is used to modify the outer profile of the actuation cell 1530. For example the internal structure 1535 can couple opposite sides of the actuation cell 1530 together to limit movement between these sections as the actuation cell 1530 is inflated. In this regard, the outer profile of the actuation cell 1530 can be controlled by incorporating one or more internal structures 1535 into the actuation cell 1530. In the illustrated example, the height of the actuation cell 1530 can be increased by limiting the movement of the opposite sides away from each other. The internal structure 1535 can be a fluid permeable structure such that the actuation cell 1530 forms a single continuous volume. In other cases, the internal structure 1535 can be fluid impermeable and used to form multiple sealed volumes within the actuation cell 1530.

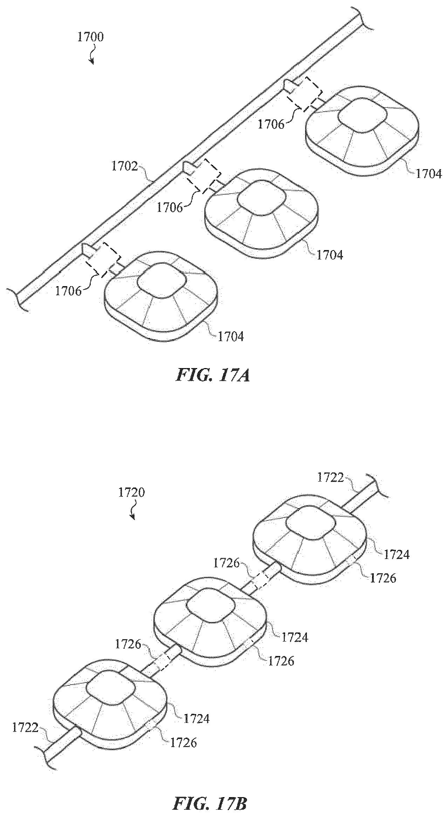

FIG. 17A shows an example of an actuation cell 1700 that can be implemented in an in-bed device as described herein. The actuation cell 1700 can include a sealed fluid passage 1702 that connects multiple inflatable bladders 1704. The positioning of the inflatable bladders 1704 within the in-bed device can be configured to create different haptic experiences for a user. For example, as illustrated in FIG. 17A, the inflatable bladders 1704 can be positioned along a first dimension that spans a width of a bed. In this regard, different ones of the bladders 1704 may be located at different positions under a user. Accordingly, when inflated or deflated the inflatable bladders 1704 can create a haptic output that is localized to specific contact regions with a user. In the example shown in FIG 17A, the multiple bladders 1704 can be coupled to the fluid passage in a parallel configuration. In some cases, an optional valve and/or pump 1706 can be positioned between each inflatable cell 1704 and the fluid passage, which can be used to independently inflate or deflate each of the inflatable bladders. For example, the fluid passage 1702 can be selectively coupled to a high pressure and/or low pressure reservoir as described herein, and each valve 1706 can be controlled to independently inflate or deflate each bladder 1704.

In some embodiments, the actuation cell 1700 and fluid passage 1702 defines a single sealed volume that is coupled with a pump and/or reservoir via a valve. In other cases, multiple valves and/or pumps 1706 can be coupled to various location of the actuation cell 1700. For example, each inflatable bladder 1704 can include a valve 1706, which can be used to increase a deflation rate of the actuation cell 1700. In some cases, operation of the multiple valves 1706 can be coordinated by a control system to allow some of the inflatable bladders 1704 to deflate at different times and/or rates than other ones of the inflatable bladders 1704. For example, a processing unit can be configured to control the multiple valves 1706 to deflate individual bladders 1724 in a defined sequence. In this regard, distinct haptic outputs can be created based on the order or speed at which different inflatable bladders 1704 are inflated or deflated in relation to each other. In further examples, each inflatable bladder can be individually controlled, for example, using a dedicated valve that controls air flow to and from the bladder as described herein. Individually controlling inflation or deflation of the individual bladders 1704 using different valves 1706 can increase the deflation rate of the actuation cell 1700, which may produce a more pronounced or sharper haptic response for a user.

FIG. 17B shows another example of an actuation cell 1720 that can be implemented in an in-bed devices as described herein. The actuation cell 1720 can include multiple inflatable bladders 1724 that are coupled together by one or more fluid passages 1722. In the example shown in FIG. 17B the inflatable bladders 1720 can be coupled in a series configuration. In some cases, all of the inflatable bladders 1720 can form a continuous volume such that they are inflated and/or deflated in unison. In other examples, one or more valves 1726 can be coupled to individual inflatable bladders 1724 and/ or between various one of the inflatable bladders 1724. In these cases, inflation and deflation of different inflatable bladders 1724 or groups of inflatable bladders 1724 can be controlled via the valves. For example, a processing unit can be configured to control the multiple valves 1726 to deflate individual bladders 1724 in a defined sequence. In this regard, different sequences of inflation or deflation can be performed to create different haptic outputs.

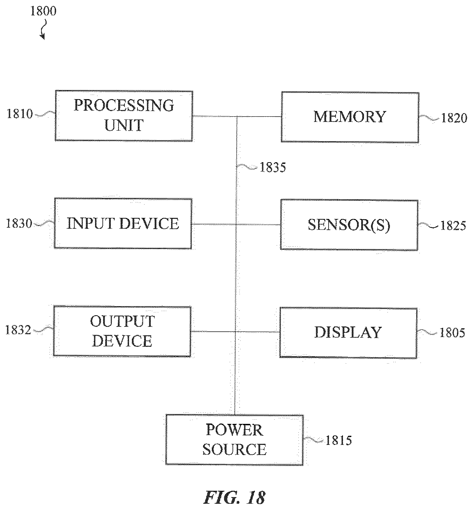

FIG. 18 shows a sample electrical block diagram of an electronic device 1800 that may incorporate and/or be connected to an in-bed haptic device. The electronic device may in some cases take the form of any suitable electronic device, including in-bed haptic devices as described herein, sleep monitors, wearable electronic devices, timekeeping devices, health monitoring or fitness devices, portable computing devices, mobile phones (including smart phones), tablet computing devices, digital media players, virtual reality devices, audio devices (including earbuds and headphones), and the like. The electronic device 1800 can include a display 1805 (e.g., a light-emitting display), a processing unit 1810, a power source 1815, a memory 1820 or storage device, a sensor 1825, an input device 1830, and an output device 1832 (e.g., an in-bed haptic device).

The processing unit 1810 can control some or all of the operations of the electronic device 1800. The processing unit 1810 can communicate, either directly or indirectly, with some or all of the components of the electronic device 1800. For example, a system bus or other communication mechanism 1835 can provide communication between the processing unit 1810, the power source 1815, the memory 1820, the sensor 1825, and the input device(s) 1830 and the output device(s) 1832.

The processing unit 1810 can be implemented as any electronic device capable of processing, receiving, or transmitting data or instructions. For example, the processing unit 1810 can be a microprocessor, a central processing unit (CPU), an application-specific integrated circuit (ASIC), a digital signal processor (DSP), or combinations of such devices. As described herein, the term "processing unit" is meant to encompass a single processor or processing unit, multiple processors, multiple processing units, or other suitably configured computing element or elements.

It should be noted that the components of the electronic device 1800 can be controlled by multiple processing units. For example, select components of the electronic device 1800 (e.g., a sensor 1825) may be controlled by a first processing unit and other components of the electronic device 1800 (e.g., the display 1805) may be controlled by a second processing unit, where the first and second processing units may or may not be in communication with each other. In some cases, the processing unit 1810 may determine a biological parameter of a user of the electronic device, such as an ECG for the user.

The power source 1815 can be implemented with any device capable of providing energy to the electronic device 1800. For example, the power source 1815 may be one or more batteries or rechargeable batteries. Additionally or alternatively, the power source 1815 can be a power connector or power cord that connects the electronic device 1800 to another power source, such as a wall outlet.

The memory 1820 can store electronic data that can be used by the electronic device 1800. For example, the memory 1820 can store electrical data or content such as, for example, audio and video files, documents and applications, device settings and user preferences, timing signals, control signals, and data structures or databases. The memory 1820 can be configured as any type of memory. By way of example only, the memory 1820 can be implemented as random access memory, read-only memory, Flash memory, removable memory, other types of storage elements, or combinations of such devices.

The electronic device 1800 may also include one or more sensors 1825 positioned almost anywhere on the electronic device 1800. The sensor(s) 1825 can be configured to sense one or more type of parameters, such as but not limited to, pressure, light, touch, heat, movement, relative motion, biometric data (e.g., biological parameters), and so on. For example, the sensor(s) 1825 may include a heat sensor, a position sensor, a light or optical sensor, an accelerometer, a pressure transducer, a gyroscope, a magnetometer, a health monitoring sensor, and so on. Additionally, the one or more sensors 1825 can utilize any suitable sensing technology, including, but not limited to, capacitive, ultrasonic, resistive, optical, ultrasound, piezoelectric, and thermal sensing technology. In some examples, the sensors 1825 may include one or more of the contact sensors, force sensors (e.g., pressure transducers), and/or electrodes described herein (e.g., one or more electrodes in a layered sensor as described herein).

In various embodiments, the display 1805 provides a graphical output, for example associated with an operating system, user interface, and/or applications of the electronic device 1800. In one embodiment, the display 1805 includes one or more sensors and is configured as a touch-sensitive (e.g., single-touch, multi-touch) and/or force-sensitive display to receive inputs from a user. For example, the display 1805 may be integrated with a touch sensor (e.g., a capacitive touch sensor) and/or a force sensor to provide a touch- and/or force-sensitive display. The display 1805 is operably coupled to the processing unit 1810 of the electronic device 1800.

The display 1805 can be implemented with any suitable technology, including, but not limited to liquid crystal display (LCD) technology, light emitting diode (LED) technology, organic light-emitting display (OLED) technology, organic electroluminescence (OEL) technology, or another type of display technology. In some cases, the display 1805 is positioned beneath and viewable through a cover sheet that forms at least a portion of an enclosure of the electronic device 1800.

In various embodiments, the input devices 1830 may include any suitable components for detecting inputs. Examples of input devices 1830 include audio sensors (e.g., microphones), optical or visual sensors (e.g., cameras, visible light sensors, or invisible light sensors), proximity sensors, touch sensors, force sensors, mechanical devices (e.g., crowns, switches, buttons, or keys), vibration sensors, orientation sensors, motion sensors (e.g., accelerometers or velocity sensors), location sensors (e.g., global positioning system (GPS) devices), thermal sensors, communication devices (e.g., wired or wireless communication devices), resistive sensors, magnetic sensors, electroactive polymers (EAPs), strain gauges, electrodes, and so on, or some combination thereof. Each input device 1830 may be configured to detect one or more particular types of input and provide a signal (e.g., an input signal) corresponding to the detected input. The signal may be provided, for example, to the processing unit 1810.

As discussed above, in some cases, the input device(s) 1830 include a touch sensor (e.g., a capacitive touch sensor) integrated with the display 1805 to provide a touch-sensitive display. Similarly, in some cases, the input device(s) 1830 include a force sensor (e.g., a capacitive force sensor) integrated with the display 1805 to provide a force-sensitive display.

The output devices 1832 may include any suitable components for providing outputs. Examples of output devices 1832 include in-bed haptic devices discussed herein, audio output devices (e.g., speakers), visual output devices (e.g., lights or displays), tactile output devices (e.g., haptic output devices), communication devices (e.g., wired or wireless communication devices), and so on, or some combination thereof. Each output device 1832 may be configured to receive one or more signals (e.g., an output signal provided by the processing unit 1810) and provide an output corresponding to the signal.

In some cases, input devices 1830 and output devices 1832 are implemented together as a single device. For example, an input/output device or port can transmit electronic signals via a communications network, such as a wireless and/or wired network connection. Examples of wireless and wired network connections include, but are not limited to, cellular, Wi-Fi, Bluetooth, IR, and Ethernet connections.

The processing unit 1810 may be operably coupled to the input devices 1830 and the output devices 1832. The processing unit 1810 may be adapted to exchange signals with the input devices 1830 and the output devices 1832. For example, the processing unit 1810 may receive an input signal from an input device 1830 that corresponds to an input detected by the input device 1830. The processing unit 1810 may interpret the received input signal to determine whether to provide and/or change one or more outputs in response to the input signal. The processing unit 1810 may then send an output signal to one or more of the output devices 1832, to provide and/or change outputs as appropriate.

As described above, one aspect of the present technology is the gathering and use of data available from various sources to provide haptic feedback, and the like. The present disclosure contemplates that in some instances, this gathered data may include personal information data that uniquely identifies or can be used to contact or locate a specific person. Such personal information data can include demographic data, location-based data, telephone numbers, email addresses, twitter ID's, home addresses, data or records relating to a user's health or level of fitness (e.g., vital signs measurements, medication information, exercise information), date of birth, or any other identifying or personal information.

The present disclosure recognizes that the use of such personal information data, in the present technology, can be used to the benefit of users. For example, the personal information data can be used to provide haptic outputs that are tailored to the user. Further, other uses for personal information data that benefit the user are also contemplated by the present disclosure. For instance, health and fitness data may be used to provide insights into a user's general wellness, or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining personal information data private and secure. Such policies should be easily accessible by users, and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and adapted to applicable laws and standards, including jurisdiction-specific considerations. For instance, in the US, collection of or access to certain health data may be governed by federal and/or state laws, such as the Health Insurance Portability and Accountability Act (HIPAA); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of haptic outputs, the present technology can be configured to allow users to select to "opt in" or "opt out" of participation in the collection of personal information data during registration for services or anytime thereafter. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, haptic outputs may be provided based on non-personal information data or a bare minimum amount of personal information, such as events or states at the device associated with a user, other non-personal information, or publicly available information.

The foregoing description, for purposes of explanation, uses specific nomenclature to provide a thorough understanding of the described embodiments. However, it will be apparent to one skilled in the art that the specific details are not required in order to practice the described embodiments. Thus, the foregoing descriptions of the specific embodiments described herein are presented for purposes of illustration and description. They are not targeted to be exhaustive or to limit the embodiments to the precise forms disclosed. It will be apparent to one of ordinary skill in the art that many modifications and variations are possible in view of the above teachings.

## Claims

1. An in-bed haptic system, comprising:
a sensing pad configured for placement between a user and a bed and comprising:
an array of actuation cells, each actuation cell in the array of actuation cells configured to actuate in response to a fluid being introduced into each actuation cell; and
an array of pressure sensors, each pressure sensor in the array of pressure sensors configured to output pressure measurements; and
a control system configured to:
receive the pressure measurements from the array of pressure sensors;
generate a pressure map for the user based on the pressure measurements and a position of each pressure sensor on the sensing pad; and
actuate actuation cells of the array of actuation cells based on the pressure map.

2. The in-bed haptic system of claim 1, wherein:
each pressure sensor in the array of pressure sensors is associated with an actuation cell in the array of actuation cells and is configured to sense a fluid pressure inside a corresponding actuation cell;
the control system is configured to pressurize individual cells of the array of actuation cells; and
the control system is configured to receive the pressure measurements taken during the pressurization of the individual cells.

3. The in-bed haptic system of claim 1, wherein the control system is configured to:
compare the pressure map to a set of stored pressure map data; and
determine a posture of the user based on the comparison.

4. The in-bed haptic system of claim 3, wherein the set of stored pressure map data comprises pressure maps corresponding to different postures of the user.

5. The in-bed haptic system of claim 1, wherein the control system is configured to:
compare the pressure map to a set of stored pressure map data; and
determine a specific user from a set of one or more users based on the comparison.

6. The in-bed haptic system of claim 1, wherein the control system is configured to:
determine a subset of the array of actuation cells that are positioned underneath the user; and
introduce pressurized air into each of the subset of the array of actuation cells to provide a haptic output.

7. The in-bed haptic system of claim 1, wherein the control system is configured to:
determine a subset of the array of actuation cells that are positioned underneath the user; and
determine a physiological parameter of the user using pressure sensors from the array of pressure sensors that correspond to the subset of the array of actuation cells.

8. The in-bed haptic system of claim 1, wherein the control system is configured to:
determine a subset of the array of actuation cells that are not being contacted by the user; and
deactivate pressure sensors associated with the subset of the array of actuation cells.

9. A pressure mapping system, comprising:
a sensing pad configured to be integrated with a user support structure and comprising:
an array of actuation cells, each actuation cell in the array of actuation cells configured to actuate in response to a fluid being introduced into an actuation cell; and
an array of pressure sensors, each pressure sensor in the array of pressure sensors coupled with the actuation cell of the array of actuation cells, configured to measure a fluid pressure within a corresponding actuation cell, and output one or more signals indicative of the measured fluid pressure for each actuation cell; and
a control system configured to:
receive the one or more signals from the array of pressure sensors;
determine that a user is contacting the user support structure;
in response to determining the user contact, generate a pressure map for the user based on the received one or more signals; and
identify the user as a specific user based on the pressure map.

10. The pressure mapping system of claim 9, wherein the control system is configured to identify the user by comparing the pressure map to a set of registered pressure maps each associated with the specific user.

11. The pressure mapping system of claim 10, wherein the control system is further configured to:
compare the pressure map to a set of registered posture maps associated with the user; and
determine a posture of the user based on the comparison of the pressure map to the set of registered pressure maps.

12. The pressure mapping system of claim 9, wherein the control system is further configured to:
identify a subset of pressure sensors from the array of pressure sensors that are positioned underneath the user; and
operate the subset of pressure sensors to measure a physiological parameter of the user.

13. The pressure mapping system of claim 9, wherein:
pressure sensors in the array of pressure sensors comprise a first sensor type;
the sensing pad further comprises a second array of sensors comprising a second sensor type, different from the first sensor type; and
the control system is configured to operate the second array of sensors to generate a second map for the user based on outputs from the second array of sensors.

14. The pressure mapping system of claim 13, wherein the control system is further configured to:
identify a subset of sensors from the second array of sensors that are positioned underneath the user; and
operate the subset of sensors from the second array of sensors to measure a physiological parameter of the user.

15. The pressure mapping system of claim 13, wherein the second sensor type comprises at least one of a temperature sensor, an acoustic sensor, a proximity sensor, a resonant sensor, a strain sensor, or a combination thereof.

16. A method of operating a pressure sensing haptic device to identify a posture of a user, the method comprising:
receiving a set of pressure measurements from an array of pressure sensors positioned on a user support structure;
generating a pressure map for the user based on the set of pressure measurements and positions of pressure sensors within the array of pressure sensors;
comparing the pressure map to a set of stored pressure maps, the set of stored pressure maps comprising posture maps that are each associated with a specific posture; and
identifying the posture of the user based on the comparison of the pressure map to the set of stored pressure maps.

17. The method of claim 16, further comprising:
identifying a set of actuators positioned under the user based on the pressure map; and
in response to identifying the set of actuators, causing the pressure sensing haptic device to actuate one or more actuators in the set of actuators to provide a haptic feedback to the user.

18. The method of claim 17, wherein the causing the pressure sensing haptic device to actuate the one or more actuators comprises causing the pressure sensing haptic device to inflate a set of actuation bladders coupled to the pressure sensing haptic device.

19. The method of claim 16, further comprising:
identifying a set of sensors positioned under the user based on the pressure map; and
in response to identifying the set of sensors, causing one or more sensors in the set of sensors to measure a physiological parameter of the user.

20. The method of claim 16, further comprising prior to receiving the set of pressure measurements, causing the pressure sensing haptic device to inflate a set of actuation bladders coupled to the pressure sensing haptic device, wherein the set of pressure measurements comprises fluid pressure measurements within the set of actuation bladders.
